# EUROPEAN PATENT APPLICATION

(11) **EP 4 332 106 A1**
(43) Date of publication of application: **06.03.2024**
(21) Application number: 22828728.0
(22) Date of filing: 21.06.2022
(51) Int. Cl.: C07D 513/04, A61K 31/444, A61K 31/506, C07D 487/04, C07D 401/12, C07D 471/04, C07D 401/14, C07D 417/12, C07D 403/12

(54) **NOVEL COMPOUND AS PROTEIN KINASE INHIBITOR**

(30) Priority: 22.06.2021 KR 20210080516
(71) Applicant: LG Chem, Ltd., Yeongdeungpo-gu Seoul 07336 (KR)
(72) Inventor: HAM, Young Jin, Daejeon 34122 (KR); CHO, Joong Heui, Daejeon 34122 (KR)
(74) Representative: Hoffmann Eitle
(86) International application number: PCT/KR2022/008812
(87) International publication number: WO 2022/270881

(57) **Abstract**

The present invention relates to a novel compound as a protein kinase inhibitor.

## Description

### TECHNICAL FIELD

### Cross-reference to Related Applications

This application claims the benefit of the priority of Korean Patent Application No. 10-2021-0080516, filed on June 22, 2021, the disclosure of which is incorporated herein in its entirety by reference.

### Technical Field

The present invention relates to a novel compound as a protein kinase inhibitor. Specifically, the present invention relates to a novel compound as a C-MET and/or RON inhibitor.

### BACKGROUND

At least 400 protein kinases that catalyze a phosphoryl transfer reaction from adenosine triphosphate (ATP) to a protein substrate are known. In a target protein to which the phosphoryl group is transferred, a specific amino acid is tyrosine, serine, or threonine, so that protein kinases are commonly referred to as protein tyrosine kinases (PTKs) or serine/threonine kinases (STKs).

The protein kinases constitute a large family of structurally related enzymes that are responsible for the control of a wide variety of signaling pathways within the cell. The signaling pathways include many other signaling pathways by the transfer of the phosphoryl group to the target protein. These phosphorylation events act as molecular on/off switches that can regulate the biological function of the target protein or protein complex. The appropriate protein kinase functions in signaling pathways are to activate or inactivate metabolic enzymes, regulatory proteins, receptors, cytoskeletal proteins, ion channels and pumps, transcription factors, and the like. Uncontrolled signaling due to defective control of protein phosphorylation has been implicated in a number of diseases, including, inflammation, cancer, allergy/asthma, diseases of the immune system, diseases of the central nervous system, angiogenesis, and the like.

Almost all kinases contain a similar 250-300 amino acid catalytic domain. The kinases may be categorized by the substrates they phosphorylate, and sequence motifs have been identified that generally correspond to each of these kinase families.

Meanwhile, a mesenchymal-epithelial transition factor (c-MET) is also referred to as a tyrosine protein kinase receptor or hepatocyte growth factor receptor, and is expressed in endothelial, epithelial, and mesenchymal cells. c-MET has been reported to be associated with the progression of certain tumors, and overexpression of c-MET is known to occur in a variety of tumor types including colon, breast, kidney, lung, hemangioma, squamous cell myeloid leukemia, melanoma, glioblastoma and astrocytoma.

In addition, a receptor originated from nantes (RON), which is one of tyrosine protein kinase receptors, is also referred to as a macrophage stimulating 1 receptor (MST1R), and has been reported to promote the invasion and metastasis of cancer cells. Overexpression of RON is also known to appear in a variety of tumor types.

Activation of tyrosine protein kinases such as c-MET and RON in tumor cells increases the proliferation, invasion, and metastasis of tumor cells, and also increases the resistance of tumor cells to apoptosis and cytotoxic therapy. Therefore, a selective small molecule kinase modulator targeting tyrosine protein kinases such as c-MET and RON is expected to have therapeutic potential for the treatment of cancers in which activation of c-MET receptors and/or RON receptors, and the like plays a critical role in the development and progression of primary tumors and secondary metastases. Accordingly, continuous research is being conducted on various inhibitors for inhibiting the activity of tyrosine protein kinases such as c-MET receptor and/or RON.

### DETAILED DESCRIPTION

### TECHNICAL PROBLEM

An aspect of the present invention provides a novel compound having a protein kinase inhibitory activity.

Another aspect of the present invention provides a compound useful for the prevention or treatment of cancer.

Yet another aspect of the present invention provides a compound useful for the prevention or treatment of immune related diseases.

Still yet another aspect of the present invention provides a compound useful as a c-MET and/or RON inhibitor.

### TECHNICAL SOLUTION

In order to achieve the above objectives,
according to an aspect of the present invention, there is provided a compound of Formula 1 below or a pharmaceutically acceptable salt thereof.

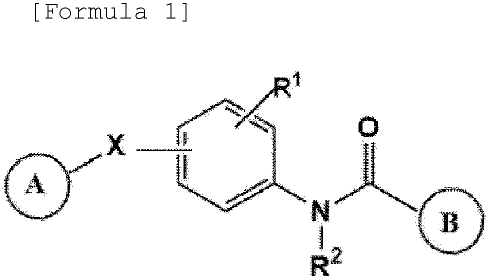

In Formula 1 above,
X above is a direct linkage, O, NR³ or NHCO,
A above is a 5- to 15-membered substituted or unsubstituted heteroaryl containing 1 to 5 hetero ring atoms selected from the group consisting of nitrogen, sulfur, and oxygen, wherein a substituent of the substituted heteroaryl is C₁-C₆ alkyl, halogen, amine, oxo, or C₂-C₆ cycloalkyl carboxamido,
A above is a single ring of the substituted or unsubstituted heteroaryl defined above, or a multi-ring in which two or more rings of the substituted or unsubstituted heteroaryl are fused or are connected by a carbon (sp²)-carbon (sp²) bond,
B above is a 5- to 7-membered substituted or unsubstituted heteroaryl containing an oxo group and containing 1 to 5 hetero ring atoms selected from the group consisting of nitrogen, sulfur and oxygen, wherein a substituent of the substituted heteroaryl is C₁-C₆ alkyl, hydroxy or halogen substituted C₁-C₆ alkyl, C₁-C₆ alkoxy, 5- to 12-membered aryl or halogen substituted 5- to 12-membered aryl,
R¹ above is hydrogen, C₁-C₆ alkyl or halogen,
R² above is hydrogen or C₁-C₆ alkyl,
R³ above is hydrogen or C₁-C₆ alkyl, and
the halogens are each independently selected from the group consisting of F, Cl, Br, and I.

According to another aspect of the present invention, there is provided a pharmaceutical composition including a compound of Formula 1 above or a pharmaceutically acceptable salt thereof, and a pharmaceutically acceptable carrier.

### ADVANTAGEOUS EFFECTS

The present invention can provide a novel compound or a pharmaceutically acceptable salt thereof which can be effectively used in the treatment of various immune related diseases, as anticancer drugs by inhibiting the activity of protein kinases of receptors, for example, c-MET and/or RON, but the effects of the present invention are not limited thereto.

### MODE FOR CARRYING OUT THE INVENTION

According to an aspect, the present invention provides a novel compound of Formula 1 as defined above or a pharmaceutically acceptable salt thereof.

In the present invention, the compound is intended to include a compound of Formula 1, a stereoisomer such as an enantiomer thereof and a diastereomer, a solvate, a prodrug, etc., unless otherwise stated.

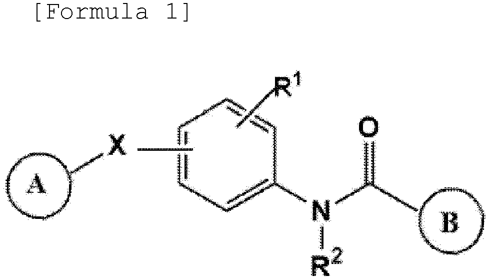

In Formula 1 above,
X above is a direct linkage, O, NR³ or NHCO,
A above is a 5- to 15-membered substituted or unsubstituted heteroaryl containing 1 to 5 hetero ring atoms selected from the group consisting of nitrogen, sulfur, and oxygen, wherein a substituent of the substituted heteroaryl is C₁-C₆ alkyl, halogen, amine, oxo, or C₂-C₆ cycloalkyl carboxamido,
A above is a single ring of the substituted or unsubstituted heteroaryl defined above, or a multi-ring in which two or more rings of the substituted or unsubstituted heteroaryl are fused or are connected by a carbon (sp²)-carbon (sp²) bond,
B above is a 5- to 7-membered substituted or unsubstituted heteroaryl containing an oxo group and containing 1 to 5 hetero ring atoms selected from the group consisting of nitrogen, sulfur and oxygen, wherein a substituent of the substituted heteroaryl is halogen, C₁-C₆ alkyl, hydroxy substituted C₁-C₆ alkyl, halogen substituted C₁-C₆ alkyl, C₁-C₆ alkyl substituted C₁-C₆ alkyl, C₁-C₆ alkoxy, 5-to 12-membered aryl, or halogen substituted 5- to 12-membered aryl,
R¹ above is hydrogen, C₁-C₆ alkyl or halogen,
R² above is hydrogen or C₁-C₆ alkyl or substituted C₁-C₆ alkyl, wherein a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea, and -SH,
R³ above is hydrogen or C₁-C₆ alkyl, and
the halogens are each independently selected from the group consisting of F, Cl, Br, and I.

In the present specification, the term "substitution" means that a hydrogen atom bonded to a carbon atom in a structure is replaced with another substituent, and the substituted position is not limited as long as it is a position at which the hydrogen atom is substituted, that is, a position at which a substituent can be substituted, and when the hydrogen atoms are substituted at two or more positions, two or more substituents may be the same as or different from each other.

In the present specification, unless otherwise defined, the "substituent" may be one or more selected from the group consisting of deuterium, halogen, hydroxy, C₁-C₁₀ alkyl, C₃-C₁₂ cycloalkyl, C₁-C₁₀ alkoxy, C₅-C₁₂ aryloxy, C₁-C₁₀ alkylthioxy, C₅-C₁₂ arylthioxy, C₁-C₁₀ alkylsulfoxy, C₅-C₁₂ arylsulfoxy, C₁-C₁₀ haloalkyl, C₂-C₂₀ alkenyl, C₀-C₁₀ amine, nitrile, nitro, imide, amide, oxo, carbonyl, carboxylic acid, carbamoyl, ester, C₅-C₁₂ aryl, and C₅-C₁₂ heteroaryl.

In the present specification, the "alkyl" may be linear or branched, and preferably has 1 to 20 carbon atoms unless otherwise defined. Examples of the alkyl include methyl, ethyl, propyl, n-propyl, isopropyl, butyl, n-butyl, isobutyl, tert-butyl, sec-butyl, 1-methylbutyl, ethylbutyl, pentyl, n-pentyl, isopentyl, neopentyl, tert-pentyl, hexyl, n-hexyl, methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, dimethylpropyl, isohexyl, 2-methylpentyl, 4-methylhexyl, 5-methylhexyl, and the like, but are not limited thereto.

In the present specification, the "cycloalkyl" may mean a cyclic saturated hydrocarbon, and preferably has 3 to 20 carbon atoms unless otherwise defined. Examples of the cycloalkyl include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, the "alkoxy" group may be linear, branched, or cyclic, and preferably has 1 to 20 carbon atoms unless otherwise defined. Examples of the alkoxy group include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the "alkenyl" group may be linear or branched, and preferably has 2 to 20 carbon atoms unless otherwise defined. Examples of the alkenyl group include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stylbenyl group, a styrenyl group, and the like, but are not limited thereto.

In the present specification, the "aryl" may be monoaryl, biaryl, or polycyclic aryl having three or more rings, and when the aryl includes two or more cyclic structures, each ring may be fused or included in a spiro form, and the aryl preferably has 5 to 12 carbon atoms unless otherwise defined. Examples of the aryl group include a phenyl group, a biphenyl group, a terphenyl group, a naphthyl group, an anthracenyl group, a phenanthryl group, a pyrenyl group, a perylenyl group, and the like, but are not limited thereto.

In the present specification, the "heteroaryl" contains at least one heteroatom other than carbon, and specifically, the heteroatom may include at least one heteroatom selected from the group consisting of O, N, Se, and S. The heteroaryl may be monocyclic or polycyclic, and when the heteroaryl includes two or more cyclic structures, each ring may be fused or included in a spiro form, and the heteroaryl preferably has 5 to 12 carbon atoms unless otherwise defined. Examples of the heteroaryl include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group, a pyridazinyl group, a pyrazinyl group, a quinolinyl group, a quinazolinyl group, a quinoxalinyl group, a phthalazinyl group, a pyridopyrimidyl group, a pyridopyrazinyl group, a pyrazinopyrazinyl group, an isoquinolinyl group, an indolyl group, a carbazolyl group, a benzoxazolyl group, a benzimidazolyl group, a benzothiazolyl group, and the like, but are not limited thereto.

According to an embodiment, in Formula 1 above, A above is a 5- to 12-membered substituted or unsubstituted heteroaryl containing 1 to 3 hetero ring atoms selected from nitrogen and sulfur, wherein the substituent of the substituted heteroaryl is halogen, amine, oxo or C₂-C₆ cycloalkyl carboxamido.

According to another embodiment, A above is a 5- to 12-membered substituted or unsubstituted heteroaryl containing 1 to 3 hetero ring atoms selected from nitrogen and sulfur, and is a single ring, or a double ring in which two heteroaryls are fused or two heteroaryls are conjugated by a carbon (sp²)-carbon (sp²) bond, wherein the substituent of the substituted heteroaryl are C₁-C₆ alkyl, halogen, amine, oxo or C₂-C₆ cycloalkyl carboxamido.

According to another embodiment, A above is pyridine, pyrimidine, pyrazole, substituted pyridine, substituted pyrimidine, or substituted pyrazole, wherein the substituent is at least one selected from a methyl group (-CH₃), an amine group (-NH₂), a chloro group (-Cl), a bromo group (-Br), and a cyclopropane carboxamido group.

According to another embodiment, A above is 9- to 12-membered heteroaryl or substituted 9- to 12-membered heteroaryl, wherein the substituent is an amine group or oxo.

According to another embodiment, A above has the following structure.

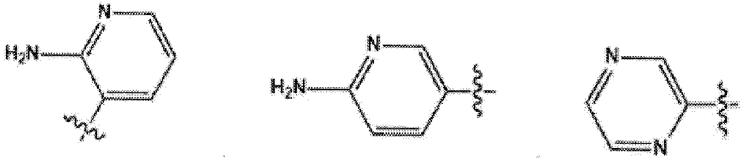

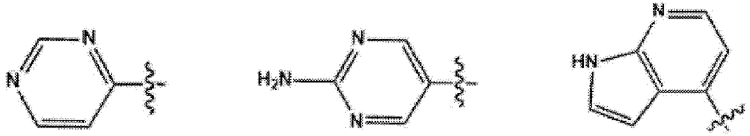

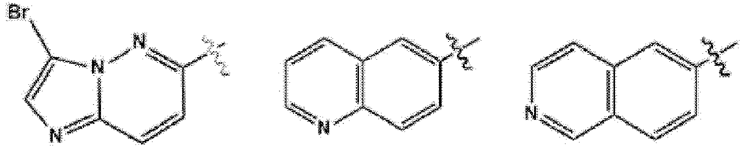

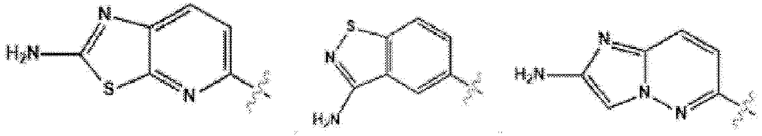

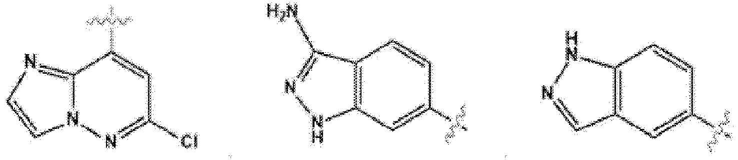

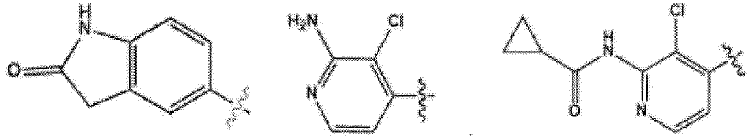

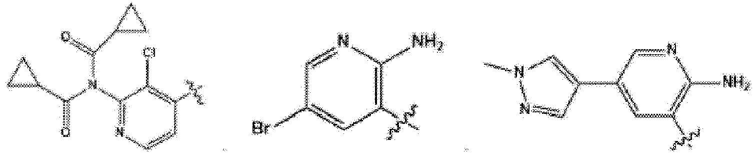

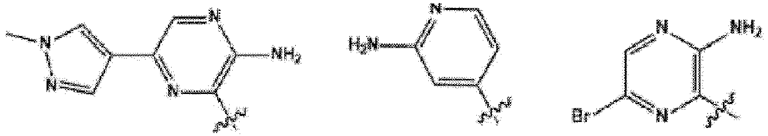

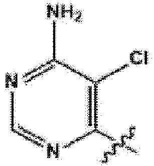

According to an embodiment, in Formula 1 above, B above has a structure of Formula 2 or Formula 3.

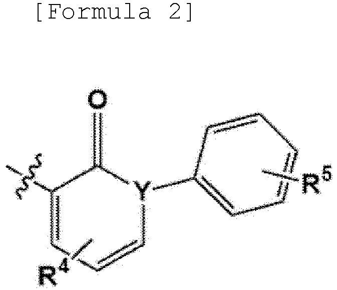

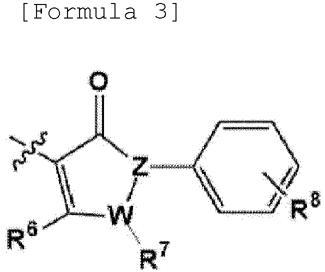

In Formula 2 or Formula 3 above,
Y, Z, and W above are each independently a heteroatom selected from the group consisting of nitrogen, sulfur, and oxygen,
R⁴, R⁶, and R⁷ above are each independently hydrogen, C₁-C₆ alkyl, halogen, hydroxy substituted C₁-C₆ alkyl, halogen substituted C₁-C₆ alkyl, C₁-C₆ alkyl substituted C₁-C₆ alkyl, or C₁-C₆ alkoxy,
R₅ and R₈ above are each independently hydrogen or halogen.

According to another embodiment, in Formula 2 or Formula 3 above,
Y, Z, and W above are nitrogen atoms,
R⁴, R⁶, and R⁷ above are each independently hydrogen, methyl, halogen, ethoxy, or buthoxy,
R₅ and R₈ above are each independently hydrogen or halogen.

According to an embodiment, in Formula 1 above, X above is a direct linkage, O, NH, or NHCO.

According to an another embodiment, in Formula 1 above, R¹ above is hydrogen, methyl or fluorine.

According to an another embodiment, in Formula 1 above, R² above is hydrogen or methyl.

According to an embodiment, the compound of Formula 1 above may be selected from the group consisting of N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide, N-(4-(2-aminothiazolo[5,4-b]pyridin-5-yl)-3-fluorophenyl)-4-ethoxy-1-(4-fluorophenyl)-2-oxo-1,2-dihydropyridine-3-carboxamide, N-(4-((2-aminothiazolo[5,4-b]pyridin-5-yl)amino)-3-fluorophenyl)-4-ethoxy-1-(4-fluorophenyl)-2-oxo-1,2-dihydropyridine-3-carboxamide, N-(4-(2-aminoimidazo[1,2-b]pyridazin-6-yl)-3-fluorophenyl)-4-ethoxy-1-(4-fluorophenyl)-2-oxo-1,2-dihydropyridine-3-carboxamide, N-(4-((6-chloroimidazo[1,2-b]pyridazin-8-yl)oxy)-3-fluorophenyl)-4-ethoxy-1-(4-fluorophenyl)-2-oxo-1,2-dihydropyridine-3-carboxamide, N-(4-((6-chloroimidazo[1,2-b]pyridazin-8-yl)oxy)-3-fluorophenyl)-1-(4-fluorophenyl)-6-methyl-2-oxo-1,2-dihydropyridine-3-carboxamide, N-(3-(6-aminopyridin-3-yl)-4-methylphenyl)-4-ethoxy-1-(4-fluorophenyl)-2-oxo-1,2-dihydropyridine-3-carboxamide, N-(3-(2-aminopyrimidin-5-yl)-4-methylphenyl)-4-ethoxy-1-(4-fluorophenyl)-2-oxo-1,2-dihydropyridine-3-carboxamide, N-(3-(2-aminopyridin-4-yl)-4-methylphenyl)-4-ethoxy-1-(4-fluorophenyl)-2-oxo-1,2-dihydropyridine-3-carboxamide, N-(3-(2-aminopyridin-3-yl)-4-methylphenyl)-4-ethoxy-1-(4-fluorophenyl)-2-oxo-1,2-dihydropyridine-3-carboxamide, 4-ethoxy-1-(4-fluorophenyl)-N-(4-methyl-3-(quinolin-6-yl)phenyl)-2-oxo-1,2-dihydropyridine-3-carboxamide, 4-ethoxy-1-(4-fluorophenyl)-N-(3-(isoquinolin-6-yl)-4-methylphenyl)-2-oxo-1, 2-dihydropyridine-3-carboxamide, 4-ethoxy-1-(4-fluorophenyl)-N-(4-methyl-3-(1H-pyrrolo[2,3-b]pyridin-4-yl)phenyl)-2-oxo-1,2-dihydropyridine-3-carboxamide, N-(4-((3-bromoimidazo[1,2-b]pyridazin-6-yl)oxy)-2-fluorophenyl)-4-ethoxy-1-(4-fluorophenyl)-2-oxo-1,2-dihydropyridine-3-carboxamide, N-(4-(2-(2-aminopyrimidin-4-yl)pyridin-4-yl)-3-fluorophenyl)-1-(4-fluorophenyl)-4-methoxy-2-oxo-1,2-dihydropyridine-3-carboxamide, N-(4-(2-(2-aminopyrimidin-4-yl)pyridin-4-yl)-3-fluorophenyl)-1-(4-fluorophenyl)-6-methyl-2-oxo-1,2-dihydropyridine-3-carboxamide, N-(4-(2-(2-aminopyrimidin-4-yl)pyridin-4-yl)-3-fluorophenyl)-5-bromo-1-(4-fluorophenyl)-2-oxo-1,2-dihydropyridine-3-carboxamide, N-(4-(3-amino-1H-indazol-6-yl)-3-fluorophenyl)-4-ethoxy-1-(4-fluorophenyl)-2-oxo-1,2-dihydropyridine-3-carboxamide, 4-ethoxy-N-(3-fluoro-4-(1H-indazol-5-yl)phenyl)-1-4-fluorophenyl)-2-oxo-1, 2-dihydropyridine-3-carboxamide, 4-ethoxy-N-(3-fluoro-4-(2-oxoindolin-5-yl)phenyl)-1-(4-fluorophenyl)-2-oxo-1,2-dihydropyridine-3-carboxamide, N-(4-(3-aminobenzo[d]isothiazol-5-yl)-3-fluorophenyl)-4-ethoxy-1-(4-fluorophenyl)-2-oxo-1,2-dihydropyridine-3-carboxamide, N-(4-(3-amino-1H-indazol-6-yl)-3-fluorophenyl)-1-(2-hydroxy-2-methylpropyl)-5-methyl-3-oxo-2-phenyl-2,3-dihydro-1H-pyrazole-4-carboxamide, N-(4-(3-amino-1H-indazol-6-yl)-3-fluorophenyl)-1-(4-fluorophenyl)-6-methyl-2-oxo-1,2-dihydropyridine-3-carboxamide, N-(4-((2-amino-3-chloropyridin-4-yl)oxy)-3-fluorophenyl)-1,5-dimethyl-3-oxo-2-phenyl-2,3-dihydro-1H-pyrazole-4-carboxamide, N-(4-((2-amino-3-chloropyridin-4-yl)oxy)-3-fluorophenyl)-1-(4-fluorophenyl)-6-methyl-2-oxo-1,2-dihydropyridine-3-carboxamide, N-(4-((2-amino-3-chloropyridin-4-yl)oxy)-3-fluorophenyl)-1-(4-fluorophenyl)-6-(hydroxymethyl)-2-oxo-1,2-dihydropyridine-3-carboxamide, N-(4-((2-amino-3-chloropyridin-4-yl)oxy)-3-fluorophenyl)-5-cyclopropyl-1-(4-fluorophenyl)-2-oxo-1,2-dihydropyridine-3-carboxamide, N-(4-((2-amino-3-chloropyridin-4-yl)oxy)-3-fluorophenyl)-1-(4-fluorophenyl)-2-oxo-6-(trifluoromethyl)-1,2-dihydropyridine-3-carboxamide, N-(4-((2-amino-3-chloropyridin-4-yl)oxy)-3-fluorophenyl)-1-(2-hydroxy-2-methylpropyl)-5-methyl-3-oxo-2-phenyl-2,3-dihydro-1H-pyrazole-4-carboxamide, N-(4-((3-chloro-2-(cyclopropanecarboxamido)pyridin-4-yl)oxy)-3-fluorophenyl)-1-(4-fluorophenyl)-6-methyl-2-oxo-1,2-dihydropyridine-3-carboxamide, N-(4-((3-chloro-2-(N-(cyclopropanecarbonyl)cyclopropanecarboxamido)pyridin-4-yl)oxy)-3-fluorophenyl)-1-(4-fluorophenyl)-6-methyl-2-oxo-1,2-dihydropyridine-3-carboxamide, 2-amino-5-bromo-N-(2-fluoro-4-(1-(4-fluorophenyl)-6-methyl-2-oxo-1,2-dihydropyridin-3-carboxamido)phenyl)nicotinamide, 3-amino-6-bromo-N-(2-fluoro-4-(1-(4-fluorophenyl)-6-methyl-2-oxo-1,2-dihydropyridine-3-carboxamido)phenyl)pyrazine-2-carboxamide, 2-amino-N-(2-fluoro-4-(1-(4-fluorophenyl)-6-methyl-2-oxo-1,2-dihydropyridin-3-carboxamido)phenyl)-5-(1-methyl-1H-pyrazol-4-yl)nicotinamide, 3-amino-N-(2-fluoro-4-(1-(4-fluorophenyl)-6-methyl-2-oxo-1,2-dihydropyridin-3-carboxamido)phenyl)-6-(1-methyl-1H-pyrazol-4-yl)pyrazine-2-carboxamide and N-(4-((6-amino-5-chloropyrimidin-4-yl)oxy)-3-fluorophenyl)-1-(4-fluorophenyl)-6-methyl-2-oxo-1,2-dihydropyridine-3-carboxamide, and the structures thereof are shown in Table 1 below.

**[Table 1]**

| | **structure** | **IUPAC Name** |
|---|---|---|
| **Example 1** | 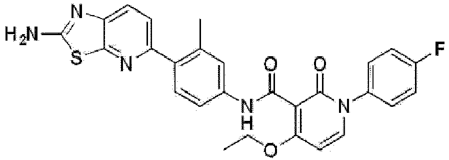 | N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide |
| **Example 2** | 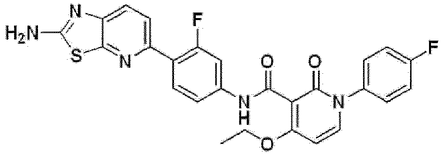 | N-(4-(2-aminothiazolo[5,4-b]pyridin-5-yl)-3-fluorophenyl)-4-ethoxy-1-(4-fluorophenyl)-2-oxo-1,2-dihydropyridine-3-carboxamide |
| **Example 3** | 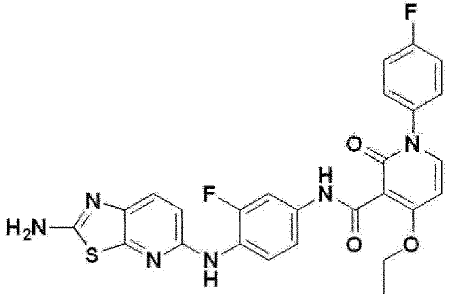 | N-(4-((2-aminothiazolo[5,4-b]pyridin-5-yl)amino)-3-fluorophenyl)-4-ethoxy-1-(4-fluorophenyl)-2-oxo-1,2-dihydropyridine-3-carboxamide |
| **Example 4** | 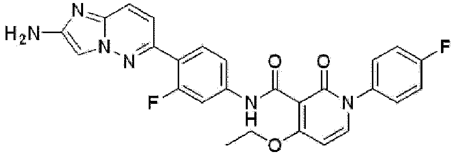 | N-(4-(2-aminoimidazo[1,2-b]pyridazin-6-yl)-3-fluorophenyl)-4-ethoxy-1-(4-fluorophenyl)-2-oxo-1,2-dihydropyridine-3-carboxamide |
| **Example 5** | 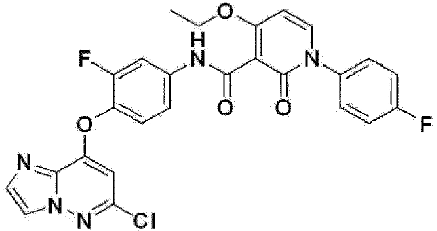 | N-(4-((6-chloroimidazo[1,2-b]pyridazin-8-yl)oxy)-3-fluorophenyl)-4-ethoxy-1-(4-fluorophenyl)-2-oxo-1,2-dihydropyridine-3-carboxamide |
| **Example 6** | 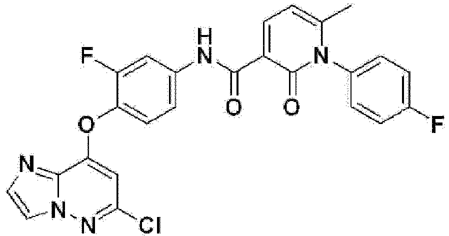 | N-(4-((6-chloroimidazo[1,2-b]pyridazin-8-yl)oxy)-3-fluorophenyl)-1-(4-fluorophenyl)-6-methyl-2-oxo-1,2-dihydropyridine-3-carboxamide |
| **Example 7** | 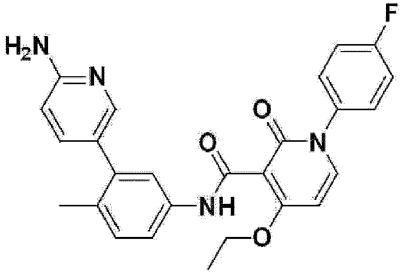 | N-(3-(6-aminopyridin-3-yl)-4-methylphenyl)-4-ethoxy-1-(4-fluorophenyl)-2-oxo-1,2-dihydropyridine-3-carboxamide |
| **Example 8** | 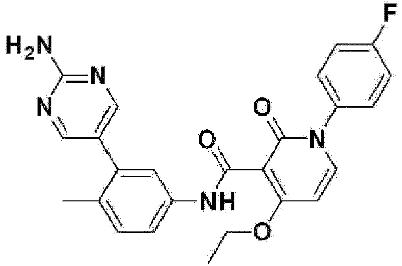 | N-(3-(2-aminopyrimidin-5-yl)-4-methylphenyl)-4-ethoxy-1-(4-fluorophenyl)-2-oxo-1,2-dihydropyridine-3-carboxamide |
| **Example 9** | 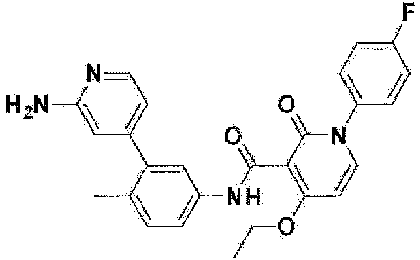 | N-(3-(2-aminopyridin-4-yl)-4-methylphenyl)-4-ethoxy-1-(4-fluorophenyl)-2-oxo-1,2-dihydropyridine-3-carboxamide |
| **Example 10** | 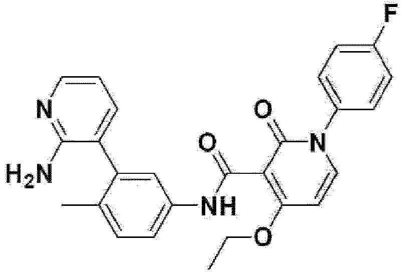 | N-(3-(2-aminopyridin-3-yl)-4-methylphenyl)-4-ethoxy-1-(4-fluorophenyl)-2-oxo-1,2-dihydropyridine-3-carboxamide |
| **Example 11** | 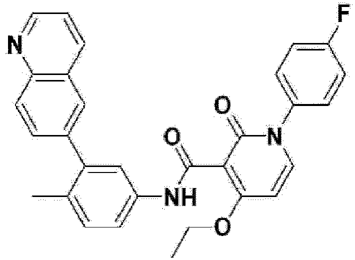 | 4-ethoxy-1-(4-fluorophenyl)-N-(4-methyl-3-(quinolin-6-yl)phenyl)-2-oxo-1,2-dihydropyridine-3-carboxamide |
| **Example 12** | 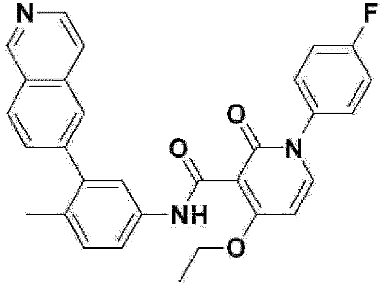 | 4-ethoxy-1-(4-fluorophenyl)-N-(3-(isoquinolin-6-yl)-4-methylphenyl)-2-oxo-1,2-dihydropyridine-3-carboxamide |
| **Example 13** | 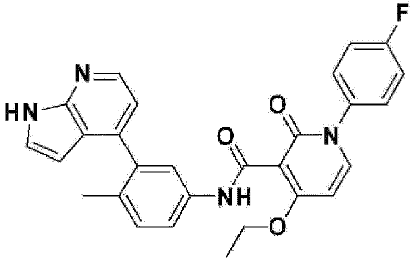 | 4-ethoxy-1-(4-fluorophenyl)-N-(4-methyl-3-(1H-pyrrolo[2,3-b]pyridin-4-yl)phenyl)-2-oxo-1,2-dihydropyridine-3-carboxamide |
| **Example 14** | 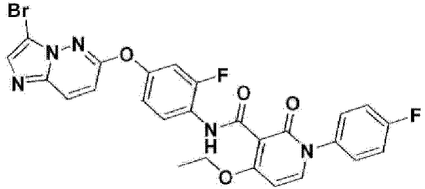 | N-(4-((3-bromoimidazo[1,2-b]pyridazin-6-yl)oxy)-2-fluorophenyl)-4-ethoxy-1-(4-fluorophenyl)-2-oxo-1,2-dihydropyridine-3-carboxamide |
| **Example 15** | 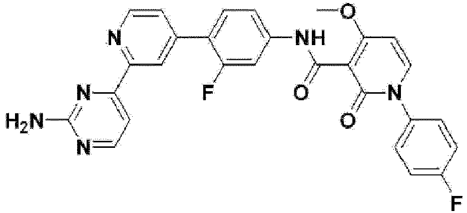 | N-(4-(2-(2-aminopyrimidin-4-yl)pyridin-4-yl)-3-fluorophenyl)-1-(4-fluorophenyl)-4-methoxy-2-oxo-1,2-dihydropyridine-3-carboxamide |
| **Example 16** | 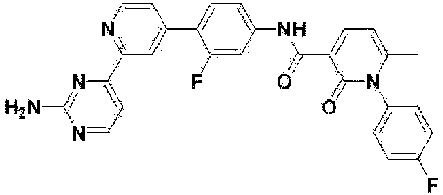 | N-(4-(2-(2-aminopyrimidin-4-yl)pyridin-4-yl)-3-fluorophenyl)-1-(4-fluorophenyl)-6-methyl-2-oxo-1,2-dihydropyridine-3-carboxamide |
| **Example 17** | 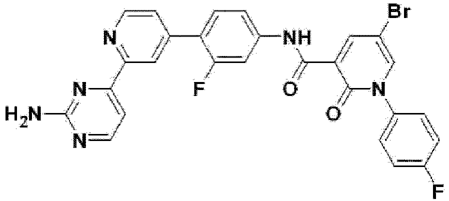 | N-(4-(2-(2-aminopyrimidin-4-yl)pyridin-4-yl)-3-fluorophenyl)-5-bromo-1-(4-fluorophenyl)-2-oxo-1,2-dihydropyridine-3-carboxamide |
| **Example 18** | 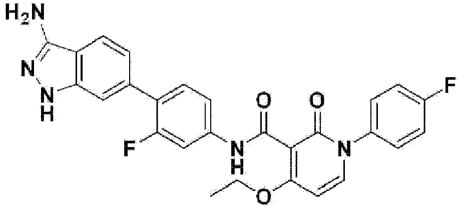 | N-(4-(3-amino-1H-indazol-6-yl)-3-fluorophenyl)-4-ethoxy-1-(4-fluorophenyl)-2-oxo-1,2-dihydropyridine-3-carboxamide |
| **Example 19** | 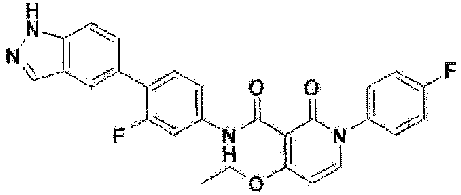 | 4-ethoxy-N-(3-fluoro-4-(1H-indazol-5-yl)phenyl)-1-4-fluorophenyl)-2-oxo-1,2-dihydropyridine-3-carboxamide |
| **Example 20** | 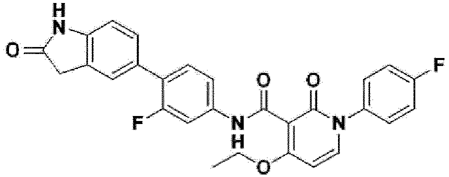 | 4-ethoxy-N-(3-fluoro-4-(2-oxoindolin-5-yl)phenyl)-1-(4-fluorophenyl)-2-oxo-1,2-dihydropyridine-3-carboxamide |
| **Example 21** | 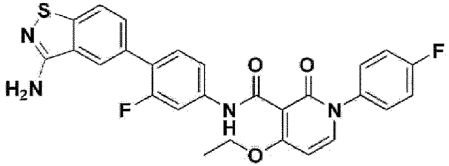 | N-(4-(3-aminobenzo[d]isoth iazol-5-yl)-3-fluorophenyl)-4-ethoxy-1-(4-fluorophenyl)-2-oxo-1,2-dihydropyridine-3-carboxamide |
| **Example 22** | 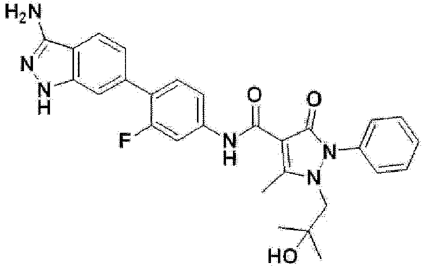 | N-(4-(3-amino-1H-indazol-6-yl)-3-fluorophenyl)-1-(2-hydroxy-2-methylpropyl)-5-methyl-3-oxo-2-phenyl-2,3-dihydro-1H-pyrazole-4-carboxamide |
| **Example 23** | 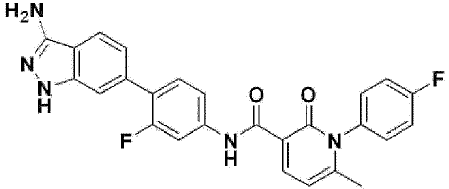 | N-(4-(3-amino-1H-indazol-6-yl)-3-fluorophenyl)-1-(4-fluorophenyl)-6-methyl-2-oxo-1,2-dihydropyridine-3-carboxamide |
| **Example 24** | 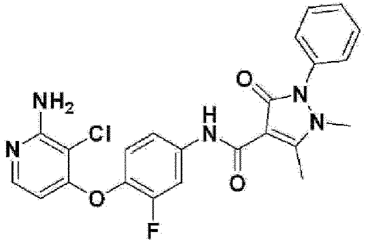 | N-(4-((2-amino-3-chloropyridin-4-yl)oxy)-3-fluorophenyl)-1,5-dimethyl-3-oxo-2-phenyl-2,3-dihydro-1H-pyrazole-4-carboxamide |
| **Example 25** | 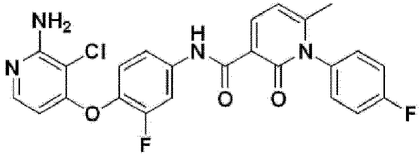 | N-(4-((2-amino-3-chloropyridin-4-yl)oxy)-3-fluorophenyl)-1-(4-fluorophenyl)-6-methyl-2-oxo-1,2-dihydropyridine-3-carboxamide |
| **Example 26** | 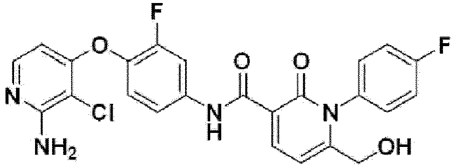 | N-(4-((2-amino-3-chloropyridin-4-yl)oxy)-3-fluorophenyl)-1-(4-fluorophenyl)-6-(hydroxymethyl)-2-oxo-1,2-dihydropyridine-3-carboxamide |
| **Example 27** | 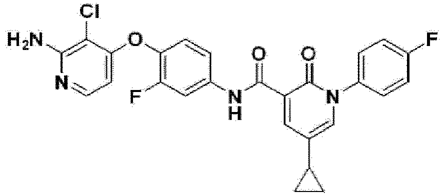 | N-(4-((2-amino-3-chloropyridin-4-yl)oxy)-3-fluorophenyl)-5-cyclopropyl-1-(4-fluorophenyl)-2-oxo-1,2-dihydropyridine-3-carboxamide |
| **Example 28** | 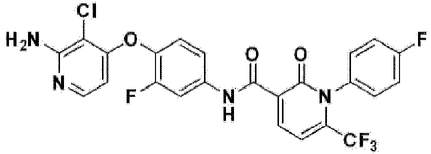 | N-(4-((2-amino-3-chloropyridin-4-yl)oxy)-3-fluorophenyl)-1-(4-fluorophenyl)-2-oxo-6-(trifluoromethyl)-1,2-dihydropyridine-3-carboxamide |
| **Example 29** | 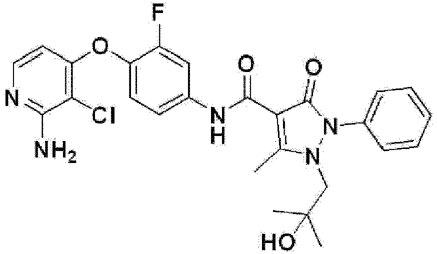 | N-(4-((2-amino-3-chloropyridin-4-yl)oxy)-3-fluorophenyl)-1-(2-hydroxy-2-methylpropyl)-5-methyl-3-oxo-2-phenyl-2,3-dihydro-1H-pyrazole-4-carboxamide |
| **Example 30** | 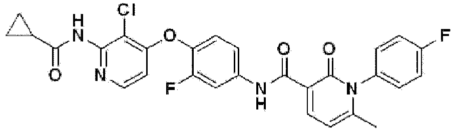 | N-(4-((3-chloro-2-(cyclopropanecarbo xamido)pyridin-4-yl)oxy)-3-fluorophenyl)-1-(4-fluorophenyl)-6-methyl-2-oxo-1,2-dihydropyridine-3-carboxamide |
| **Example 31** | 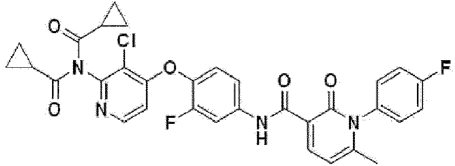 | N-(4-((3-chloro-2-(N-(cyclopropanecarbo nyl)cyclopropaneca rboxamido)pyridin-4-yl)oxy)-3-fluorophenyl)-1-(4-fluorophenyl)-6-methyl-2-oxo-1,2-dihydropyridine-3-carboxamide |
| **Example 32** | 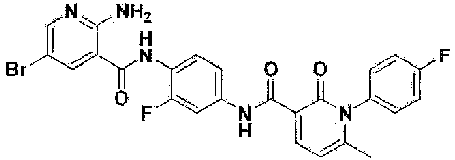 | 2-amino-5-bromo-N-(2-fluoro-4-(1-(4-fluorophenyl)-6-methyl-2-oxo-1,2-dihydropyridin-3-carboxamido)phenyl )nicotinamide |
| **Example 33** | 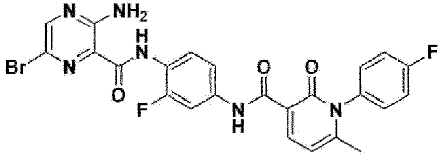 | 3-amino-6-bromo-N-(2-fluoro-4-(1-(4-fluorophenyl)-6-methyl-2-oxo-1,2-dihydropyridine-3-carboxamido)phenyl )pyrazine-2-carboxamide |
| **Example 34** | 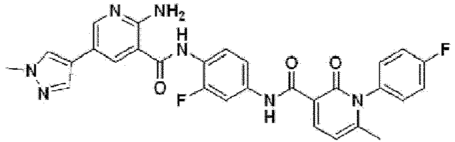 | 2-amino-N-(2-fluoro-4-(1-(4-fluorophenyl)-6-methyl-2-oxo-1,2-dihydropyridin-3-carboxamido)phenyl)-5-(1-methyl-1H-pyrazol-4-yl)nicotinamide |
| **Example 35** | 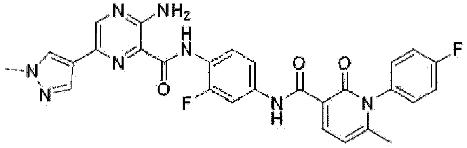 | 3-amino-N-(2-fluoro-4-(1-(4-fluorophenyl)-6-methyl-2-oxo-1,2-dihydropyridin-3-carboxamido)phenyl )-6-(1-methyl-1H-pyrazol-4-yl)pyrazine-2-carboxamide |
| **Example 36** | 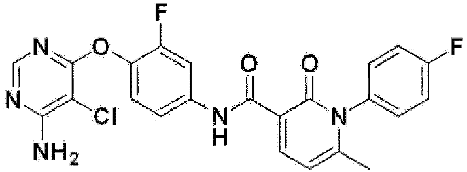 | N-(4-((6-amino-5-chloropyrimidin-4-yl)oxy)-3-fluorophenyl)-1-(4-fluorophenyl)-6-methyl-2-oxo-1,2-dihydropyridine-3-carboxamide |

In the present disclosure, the "pharmaceutically acceptable salt" includes a salt commonly used to form an alkali metal salt and to form an additional salt of a free acid or a free base. The properties of these salts are not important, but should be pharmaceutically acceptable. A suitable pharmaceutically acceptable acid addition salt of the compound of Formula 1 may be prepared from an inorganic or organic acid. Examples of such an inorganic acid include hydrochloric acid, hydrobromic acid, hydroiodic acid, nitric acid, carbonic acid, sulfuric acid, and phosphoric acid. A suitable organic acid may be selected from aliphatic, alicyclic, aromatic, arylaliphatic, heterocyclic, carboxylic and sulfonic groups of organic acids, and examples of the organic acid include formic acid, acetic acid, adipic acid, butyric acid, propionic acid, succinic acid, glycolic acid, gluconic acid, lactic acid, malic acid, tartaric acid, citric acid, ascorbic acid, glucuronic acid, maleic acid, fumaric acid, pyruvic acid, aspartic acid, glutamic acid, benzoic acid, anthranilic acid, mesylic acid, 4-hydroxybenzoic acid, phenylacetic acid, mandelic acid, embonic acid (pamoic acid), methanesulfonic acid, ethanesulfonic acid, ethanedisulfonic acid, benzenesulfonic acid, pantothenic acid, 2-hydroxyethanesulfonic acid, toluenesulfonic acid, sulfanilic acid, cyclohexylaminosulfonic acid, camphoric acid, camphorsulfonic acid, digluconic acid, cyclopentanepropionic acid, dodecylsulfonic acid, glucoheptanoic acid, glycerophosphonic acid, heptanoic acid, hexanoic acid, 2-hydroxy-ethanesulfonic acid, nicotinic acid, 2-naphthalenesulfonic acid, oxalic acid, palmoic acid, pectic acid, persulfuric acid, 2-phenylpropionic acid, picric acid, pivalic acid, propionic acid, succinic acid, tartaric acid, thiocyanic acid, mesylic acid, undecanoic acid, stearic acid, algenic acid, β-hydroxybutyric acid, salicylic acid, galactaric acid, and galacturonic acid. A suitable pharmaceutically acceptable base addition salt of the compound of Formula 1 includes metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, histidine, glucamine, isopropylamine, lysine, morpholine, N-ethyl morpholine, piperazine, piperidine, triethyl amine, and trimethyl amine. All of these salts may be prepared by conventional means from the corresponding compound of the invention by reacting, for example, the appropriate acid or base with the compound of Formula 1. When a basic group and an acid group are present in the same molecule, the compound of Formula 1 may also form internal salts.

In the present invention, the "solvate" may include a hydrate, and a solvate with an organic solvent such as methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and a mixture thereof.

According to another aspect, the present invention provides a pharmaceutical composition including a novel compound of Formula 1 defined above or a pharmaceutically acceptable salt thereof, and a pharmaceutically acceptable carrier.

The pharmaceutical composition may be useful for the prevention or treatment of protein kinase-mediated diseases, but the use of the present invention is not limited to these diseases.

In an embodiment, the pharmaceutical composition may be useful for the prevention or treatment of c-MET or RON-mediated diseases.

In an another embodiment, the pharmaceutical composition may be useful for the prevention or treatment of c-MET and RON-dual-mediated diseases.

The pharmaceutical composition according to the present invention includes a therapeutically effective amount of the compound of Formula 1 above or a pharmaceutically acceptable salt thereof.

In an embodiment, the disease may be cancer selected from the group consisting of lung cancer, breast cancer, colorectal cancer, kidney cancer, pancreatic cancer, head cancer, neck cancer, hereditary papillary renal cell carcinoma, pediatric hepatocellular carcinoma, and stomach cancer, but is not limited thereto.

In another embodiment, the disease may be an immune disease selected from the group consisting of an inflammatory disorder, a cardiovascular disease, a virus induced disease, a circulatory disease, a fibro-proliferative disease, and pain sensitization, but is not limited thereto.

For the treatment of the above-described diseases, the pharmaceutical composition may be administered to a subject in need of the prevention or treatment of the above-described diseases.

In the present specification, the term "prevention" refers to reducing the risk of developing a disease or disorder, and refers to any action that inhibits or delays the onset of a disease by preventing the progression of one or more clinical symptoms of the disease in a subject that is easily exposed to or susceptible to the disease but does not yet experience the disease or display symptoms of the disease.

In the present specification, the term "treatment" refers to alleviating a disease or disorder, and refers to any action that ameliorates or beneficially alters symptoms of the disease by arresting or reducing the progression of the disease or one or more clinical symptoms thereof.

In the present specification, the term "carrier" refers to a compound that facilitates the introduction of a compound into the cell or tissue. The pharmaceutical composition may be prepared in a unit dose form by being formulated using a pharmaceutically acceptable carrier, or may be prepared by being incorporated into a multi-dose container. In this case, the formulation may be in the form of a solution, a suspension, or an emulsion in an oil or an aqueous medium, or may be in the form of an extract, a powder, a granule, a tablet, a capsule, or a gel (e.g., a hydrogel), and may additionally include a dispersant or a stabilizer.

In addition, the compound represented by Formula 1 or the pharmaceutically acceptable salt thereof included in the pharmaceutical composition may be delivered in a pharmaceutically acceptable carrier such as colloidal suspensions, powders, saline, lipids, liposomes, microspheres, or nanospherical particles. These may be complexed with or associated with a vehicle and may be delivered in vivo using delivery systems known in the art, such as lipids, liposomes, microparticles, gold, nanoparticles, polymers, condensation reagents, polysaccharides, polyamino acids, dendrimers, saponins, adsorption enhancing substances, or fatty acids.

In addition, the pharmaceutically acceptable carrier may include lactose, dextrose, sucrose, sorbitol, mannitol, starch, acacia, rubber, calcium phosphate, alginate, gelatin, calcium silicate, microcrystalline cellulose, polyvinyl pyrolidone, cellulose, water, syrup, methyl cellulose, methyl hydroxybenzoate, propyl hydroxybenzoate, talc, magnesium stearate, mineral oil, and the like, which are typically used in formulation, but is not limited thereto. In addition, lubricants, wetting agents, sweetening agents, flavoring agents, emulsifying agents, suspending agents, preservatives, and the like may be further included in addition to the above components.

The pharmaceutical composition according to the present invention may be administered orally or parenterally at the time of clinical administration, and may be used in the form of a general pharmaceutical formulation. That is, the pharmaceutical composition of the present invention may be administered in various formulations of oral and parenteral administration at the time of actual clinical administration, and when formulated, is prepared using diluents or excipients such as fillers, extenders, binders, wetting agents, disintegrants, and surfactants, which are commonly used. Solid formulations for oral administration include tablets, pills, powders, granules, capsules, and the like, and such solid formulations are prepared by mixing a herbal medicine extract or a herbal medicine fermented product with at least one excipient, for example, starch, calcium carbonate, sucrose or lactose, gelatin, and the like. In addition to simple excipients, lubricants such as magnesium stearate and talc are also used. Liquid formulations for oral administration include suspensions, oral liquids, emulsions, syrups, and the like, and in addition to water and liquid paraffin, which are commonly used simple diluents, various excipients such as wetting agents, sweetening agents, fragrances, preservatives, and the like may be included. Formulations for parenteral administration include sterilized aqueous solutions, non-aqueous solvents, suspensions, emulsions, lyophilized formulations, and suppositories. For the non-aqueous solutions and the suspensions, propylene glycol, polyethylene glycol, vegetable oil such as olive oil, injectable ester such as ethyl oleate, or the like may be used. As the base of the suppositories, Witepsol^{™}, Macrogol, Tween^{™} 61, cacao butter, laurin fat, glycerol, gelatin, and the like may be used.

When the pharmaceutical composition is administered for a clinical purpose, the effective dosage of Formula 1 above or the pharmaceutically acceptable salt thereof may vary depending on factors such as formulation methods, administration methods, patient's age, body weight, sex, condition, and diet, time of administration, route of administration, rate of excretion, drug combination, and sensitivity of response, but may generally be 0.01 mg/day to 20 mg/day per 1 kg body weight of an adult patient, preferably 1 mg/day to 10 mg/day, and may be administered in split doses several times a day, preferably 2 to 3 times a day at regular time intervals according to the decision of a doctor or a pharmacist.

In another aspect, the present invention provides a method for treating protein kinase-mediated diseases, the method including administering to a subject a therapeutically effective amount of the compound of Formula 1 above or the pharmaceutically acceptable salt thereof.

In another aspect, the present invention provides a method for inhibiting the activity of a c-MET and/or RON receptor, the method including administering to a subject a therapeutically effective amount of the compound of Formula 1 above or the pharmaceutically acceptable salt thereof.

In the present specification, the term "therapeutically effective amount" refers to an amount of each formulation that achieves the purpose of improving the severity of a disease and frequency of onset thereof during the treatment by each formulation itself, but avoids adverse reactions typically associated with other therapies. For example, an effective therapeutic agent for tumor exhibits effects of prolonging the survival period of a patient, suppressing the proliferation of cells associated with a tumor, or regressing the tumor.

Hereinafter, example compounds of the present invention may be prepared according to the following method, but compounds of the present invention are not limited to the preparation method.

### Preparation Example 1. 4-ethoxy-1-(4-fluorophenyl)-2-oxo-1,2-dihydropyridine-3-carboxylic acid

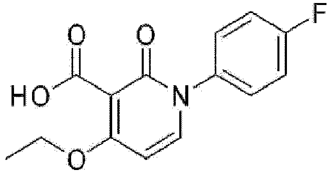

Hereinafter, a compound of Preparation Example 1 was prepared according to Reaction Scheme 1 below. 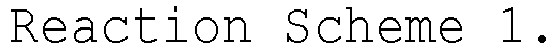 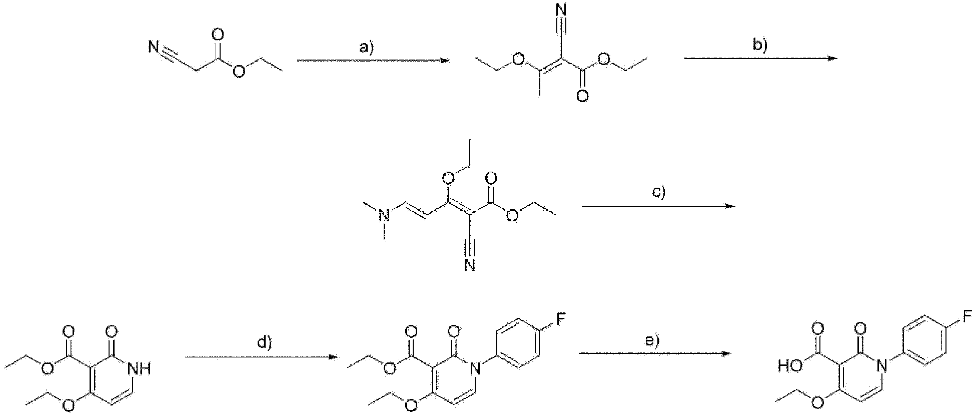 **Conditions:** a) triehtyl orthoacetate, acetic acid, 120 °C, 12 h; b) DMF-DEA, 70 °C, 2 h; c) acetic acid, 120 °C, 12 h; d) (4-fluorophenyl)boronic aicd, Cu(OAc)₂, pyridine, DCM, rt, 12 h; e) LIOH, EtOH/H₂O (2:1), rt, 12 h

### Step 1) Ethyl 4-ethoxy-2-oxo-1,2-dihydropyridine-3-carboxylate

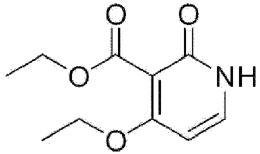

Triethyl orthoacetate (17.21 g, 106 mmol) and 50 mL of acetic acid were added to ethylcyanoacetate (6 g, 53 mmol), and the mixture was stirred at 120 °C for 12 hours. The solvent of the reaction mixture was concentrated and 7.1 mL (53 mmol) of DMF-DEA (N, N-dimethylformamide diethylacetal) was added. The reaction mixture was stirred at 70 °C for 2 hours. 50 mL of acetic acid and 6 mL of distilled water were added to the reaction mixture, and the mixture was refluxed for 12 hours. The solvent of the reaction mixture was concentrated and diluted with water. The reaction mixture was adjusted to pH 9-10 with a saturated sodium hydrogen carbonate aqueous solution and then extracted three times with DCM. The organic layer was washed with brine and concentrated. The residue was purified by column chromatography to obtain the title compound (3.6 g, yield: 32%).

¹H NMR (400 MHz, CDCl₃) δ 13.42 (brs, 1H), 7.47 (d, 1H), 6.18 (d, 1H), 4.34 (q, 2H), 4.20 (q, 2H), 1.36(m, 6H); MS m/z : 212[M+H]

### Step 2) Ethyl 4-ethoxy-1-(4-fluorophenyl)-2-oxo-1,2-dihydropyridine-3-carboxylate

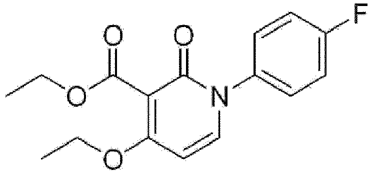

The compound ethyl 4-ethoxy-2-oxo-1,2-dihydropyridine-3-carboxylate (1.2 g, 5.68 mmol) obtained in Step 1 above was dissolved in 50 mL of DCM, and then (4-fluorophenyl)boronic acid (1.59 g, 11.4 mmol), Cu(OAc)₂ (1.75 g, 9.66 mmol), and pyridine (1.35 g, 17.04 mmol) were added thereto, followed by stirring at room temperature for 12 hours. The reaction mixture was filtered by celite, and the solids were washed with water. The filtrate was extracted twice with DCM, concentrated, and used in Step 3 below without a purification process.

MS m/z : 306 [M+H]

### Step 3) 4-ethoxy-1-(4-fluorophenyl)-2-oxo-1,2-dihydropyridine-3-carboxylic acid

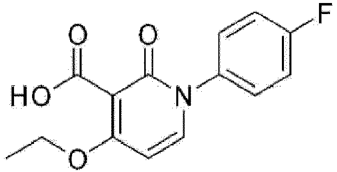

The compound ethyl 4-ethoxy-1-(4-fluorophenyl)-2-oxo-1,2-dihydropyridine-3-carboxylate obtained in Step 2 above was dissolved in an ethanol/water (2:1) mixed solution, and then LiOH (0.41 g, 17.04 mmol) was added thereto, followed by stirring at room temperature for 12 hours. The reaction mixture was concentrated, diluted with water, and washed with ethyl acetate. The aqueous solution was adjusted to pH 2 with an aqueous solution of 3N hydrogen chloride and extracted three times with DCM. The organic solution was washed with brine and concentrated. Ethyl-ether was added to the residue, and the resulting solids were filtered and dried to obtain the title compound (1.3 g, 83%).

MS m/z : 278 [M+H]

### Preparation Example 2. 4-methoxy-1-(4-fluorophenyl)-2-oxo-1,2-dihydropyridine-3-carboxylic acid

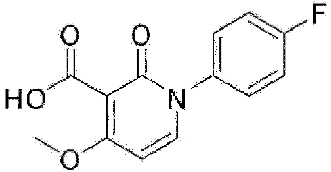

Hereinafter, a compound of Preparation Example 2 was prepared according to Reaction Scheme 2 below.

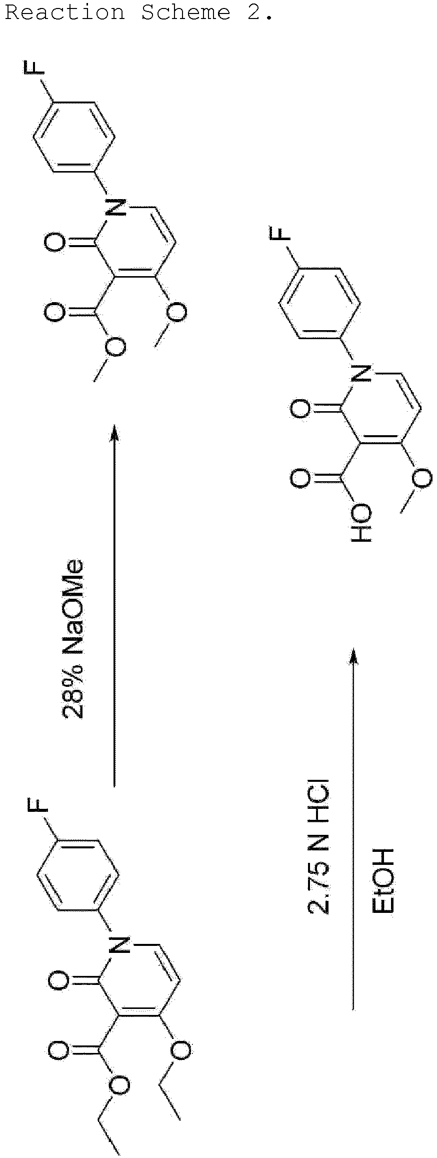

### Step 1) methyl 1-(4-fluorophenyl)-4-methoxy-2-oxo-1,2-dihydropyridine-3-carboxylate

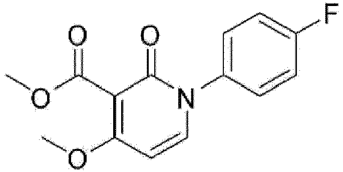

The compound ethyl 4-ethoxy-1-(4-fluorophenyl)-2-oxo-1,2-dihydropyridine-3-carboxylate (0.5 g, 1.64 mmol) obtained in Step 2 of Preparation Example 1 was treated with a 28% NaOMe solution, followed by stirring at room temperature for 10 minutes. The reaction mixture was concentrated to obtain the title compound (0.36 g, 79%).

MS m/z : 278 [M+H]

### Step 2) 4-methoxy-1-(4-fluorophenyl)-2-oxo-1,2-dihydropyridine-3-carboxylic acid

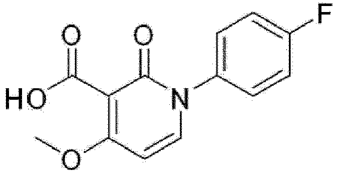

The compound methyl 1-(4-fluorophenyl)-4-methoxy-2-oxo-1,2-dihydropyridine-3-carboxylate (0.36 g, 1.29 mmol) obtained in Step 1 above was dissolved in 5 mL of ethanol, and then 5 mL of a 3N hydrochloric acid solution was added thereto at room temperature. The reaction solution was stirred at 60 °C for 4 hours, and then the resulting solids were filtered to obtain thee title compound (0.23 g, 68%).

MS m/z : 264 [M+H]

### Example 1. N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide

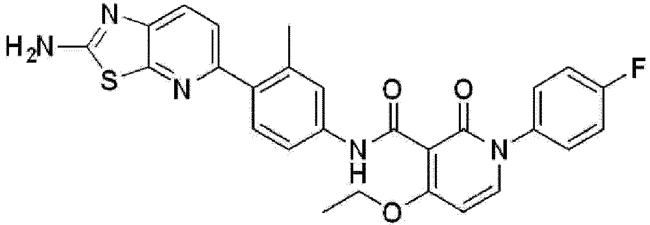

Hereinafter, a compound of Example 1 was prepared according to Reaction Scheme 3 below. 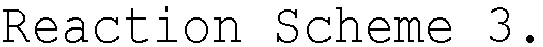 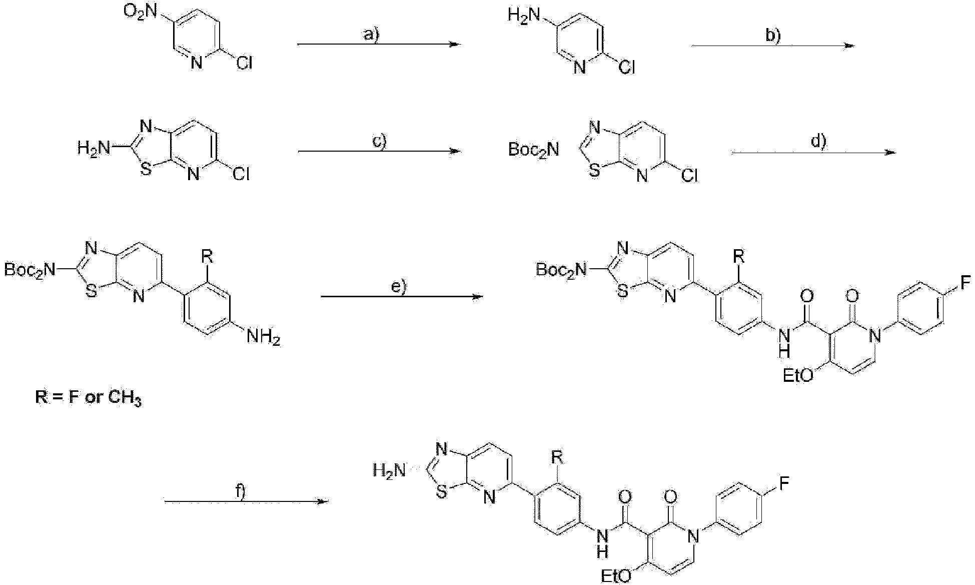 **Conditions:** a) iron, acetic acid, 80 °C, 4 h; b) KSCN, Br₂, acetic acid, rt, 12 h; c) Boc-anhydride, DMAP, THF, rt, 12 h; d) Pd(dppf)Cl₂DCM, NaHCO₃, 1,4-dioxane, 130 °C, 12 h; e) R-Acid, HATU DIPEA, THF, rt, 12 h; f) TFA, DCM, rt, 2h

### Step 1. 6-chloropyridine-3-amine

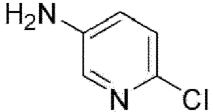

2-chloro-5-nitropyridine (5 g, 31.5 mmol) was dissolved in 50 mL of acetic acid, and iron powder (8.8 g, 158 mmol) was added thereto, followed by stirring at 80 °C for 2 hours. The reaction mixture was filtered through celite, and the filtrate was dried under vacuum. The residue was dissolved in DCM, neutralized with a saturated NaHCO₃ aqueous solution, and then extracted five times with DCM. The organic layer was washed with brine and concentrated to obtain the title compound (3.8 g, yield: 94%).

MS *m*/*z* : 129[M+H]

### Step 2. 5-chlorothiazolo[5,4-b]pyrdine-2-amine

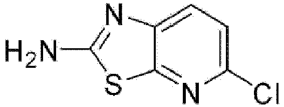

The compound 6-chloropyridine-3-amine (3.8 g, 29.5 mmol) obtained in Step 1 above and KSCN (3.06 g, 31.5 mmol) were mixed in an acetic acid solution, and bromine (1.6 ml, 31.5 mmol) was slowly added thereto, followed by stirring at room temperature for 12 hours. The reaction mixture was filtered through celite and dried under vacuum so that the volume of the filtrate was 1/3. The residue was adjusted to pH 6 with an ammonia aqueous solution, and the precipitate was filtered to obtain the title compound as a yellow solid (3.6 g, yield: 66%).

MS *m*/*z* : 186[M+H]

### Step 3. Di-t-butyl(5-chlorothiazolo[5,4-b]pyridin-2-yl)carbamate

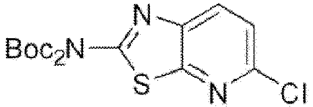

The compound 5-chlorothiazolo[5,4-b]pyridine-2-amine (2 g, 10.7 mmol) obtained in Step 2 above was dissolved in 50 mL of THF, and Boc-anhydride (5.0 mL, 21.4 mmol) and DMAP (65 mg, 0.54 mmol) were added thereto, followed by stirring at room temperature for 12 hours. The reaction was completed, and then the reaction solution was diluted with water, and extracted three times with ethyl acetate and concentrated. The residue was purified by silica gel column chromatography to obtain the title compound (3.7 g, yield: 90%).

MS *m*/*z* : 386[M+H]

### Step 4. Di-t-butyl(5-(4-amino-2-methylphenyl)thiazolo[5,4-b]pyridin-2-yl)carbamate

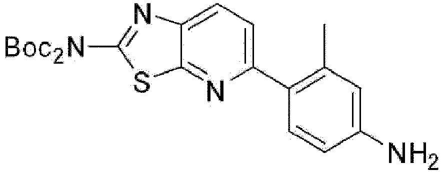

The compound di-tert-butyl(5-chlorothiazolo[5,4-b]pyridin-2-yl)carbamate (50 mg, 0.13 mmol) obtained in Step 3 above was dissolved in 2 mL of 1,4-dioxane, and then 3-methyl-4-(4,4,5,5-tetramethyl-1,3,2-dioxaborolan-2-yl)aniline (37 mg, 0.16 mmol), a saturated NaHCO₃ aqueous solution (1.5 ml, 0.07 mmol), and PdCl₂(dppf)DCM adduct (5.3 mg, 0.007 mmol) were added thereto, followed by stirring at 130 °C for 2 hours. The reaction mixture was filtered through celite, and the filtrate was dried under vacuum. The residue was purified by silica gel column chromatography to obtain the title compound (20 mg, yield: 34%).

MS *m*/*z* : 457[M+H]

### Step 5. Di-t-butyl-N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide

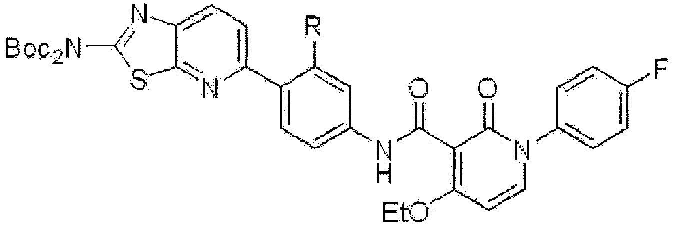

The compound di-tert-butyl(5-(4-amino-2-methylphenyl)thiazolo[5,4-b]pyridin-2-yl)carbamate (20 mg, 0.04 mmol) obtained in Step 4 above was dissolved in 2 mL of THF, and then 4-ethoxy-1-(4-fluorophenyl)-2-oxo-1,2-dihydropyridine-3-carboxylic acid (15 mg, 0.05 mmol), HATU (25 mg, 0.07 mmol), and DIPEA (12 µL, 0.07 mmol) were added thereto, followed by stirring at room temperature for 12 hours. The reaction solution was diluted with water, and then extracted three times with ethyl acetate and concentrated. The residue was purified by silica gel column chromatography to obtain the title compound (15 mg, 48%).

MS *m*/*z* : 715[M+H]

### Step 6. N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide

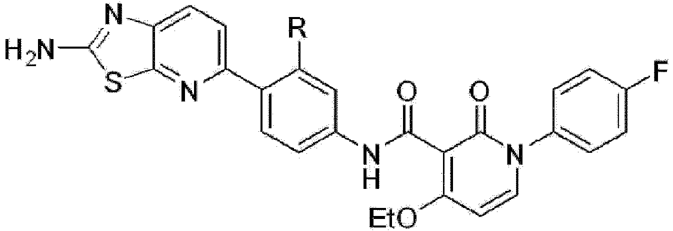

The compound di-tert-butyl-N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide (15 mg, 0.02 mmol) obtained in Step 5 above was dissolved in 1.5 mL of DCM, and then 1.5 mL of TFA was added thereto, followed by stirring at room temperature for 2 hours. The reaction solution was dried under vacuum. The residue was dissolved in DCM, then neutralized with a saturated NaHCO₃ aqueous solution, and extracted three times with DCM and concentrated. The residue was purified by prep-HPLC (0.1% formic acid in water/acetonitrile) to obtain the title compound (10 mg, yield: 93%) .

¹H NMR (400 MHz, DMSO-d₆) δ 7.78 (d, 1H), 7.74 (d, 1H), 7.63 (m, 2H), 7.47 (m, 2H), 7.38 (m, 2H), 7.31 (t, 2H), 6.60 (d, 1H), 4.35 (q, 2H), 2.36 (s, 3H), 1.47(t, 3H); MS m/z : 516[M+H]

### Example 2. N-(4-(2-aminothiazolo[5,4-b]pyridin-5-yl)-3-fluorophenyl)-4-ethoxy-1-(4-fluorophenyl)-2-oxo-1,2-dihydropyridine-3-carboxamide

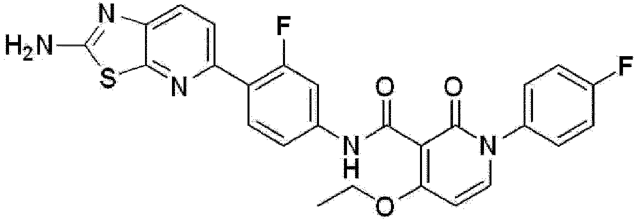

The title compound (4 mg, yield: 71%) was obtained in a manner similar to that of Example 1 using 3-fluoro-4-(4,4,5,5-tetramethyl-1,3,2-dioxaborolan-2-yl)aniline instead of 3-methyl-4-(4,4,5,5-tetramethyl-1,3,2-dioxaborolan-2-yl)aniline according to Step 4 of Example 1.

¹H NMR (400 MHz, DMSO-d₆) δ 10.6 (s, 1H), 7.91 (m, 3H), 7.79 (d, 1H), 7.69 (m, 2H), 7.45 (m, 2H), 7.36 (m, 2H), 6.52 (m, 1H), 4.25 (q, 2H), 1.30(t, 3H); MS *m*/*z* : 520[M+H]

### Example 3. N-(4-((2-aminothiazolo[5,4-b]pyridin-5-yl)amino)-3-fluorophenyl)-4-ethoxy-1-(4-fluorophenyl)-2-oxo-1,2-dihydropyridine-3-carboxamide

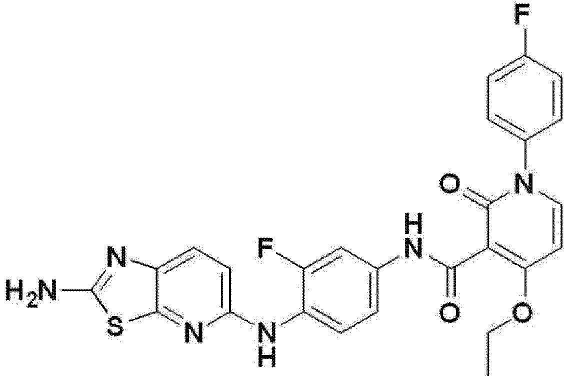

The title compound (10 mg, yield: 36%) was obtained in a manner similar to that of Example 1 using t-butyl(4-amino-3-fluorophenyl)carbamate and Pd₂(dba)₃, Xphos, and Cs₂CO₃ according to Step 4 of Example 1.

¹H NMR (400 MHz, DMSO-d₆) δ 7.98 (t, 1H), 7.76 (m, 2H), 7.53 (d, 1H), 7.46 (m, 2H), 7.29 (m, 3H), 6.82 (d, 1H), 6.58 (m, 1H), 4.34 (q, 2H), 1.46(t, 3H); MS *m*/*z* : 535[M+H]

### Example 4. N-(4-(2-aminoimidazo[1,2-b]pyridazin-6-yl)-3-fluorophenyl)-4-ethoxy-1-(4-fluorophenyl)-2-oxo-1,2-dihydropyridine-3-carboxamide

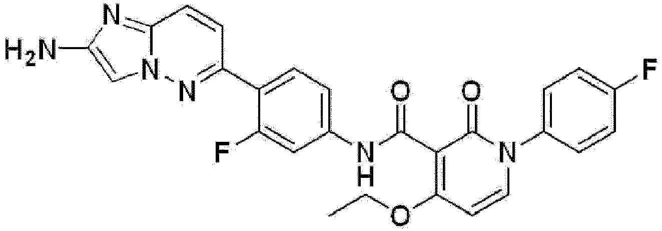

The title compound (1 mg, yield: 20%) was obtained in a manner similar to that of Example 1 using t-butyl(6-chloroimidazo[1,2-b]pyridazin-2-yl)carbamate instead of di-t-butyl(5-chlorothiazolo[5,4-b]pyridin-2-yl)carbamate according to Step 4 of Example 1.

¹H NMR (400 MHz, DMSO-d₆) δ 7.96 (m, 2H), 7.82 (d, 1H), 7.68 (d, 2H), 7.48 (m, 4H), 7.28 (m, 2H), 6.64 (d, 1H), 4.36 (q, 2H), 1.42(t, 3H);

MS m/z : 503[M+H]

### Example 5. N-(4-((6-chloroimidazo[1,2-b]pyridazin-8-yl)oxy)-3-fluorophenyl)-4-ethoxy-1-(4-fluorophenyl)-2-oxo-1,2-dihydropyridine-3-carboxamide

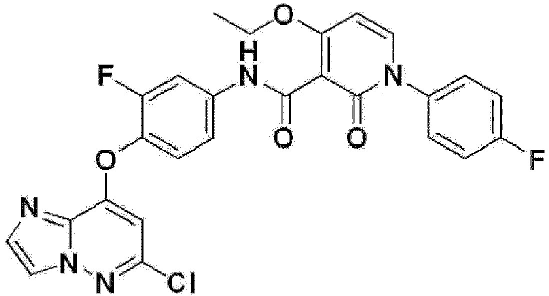

Hereinafter, a compound of Example 5 was prepared according to Reaction Scheme 4 below.

Reaction Scheme 4.

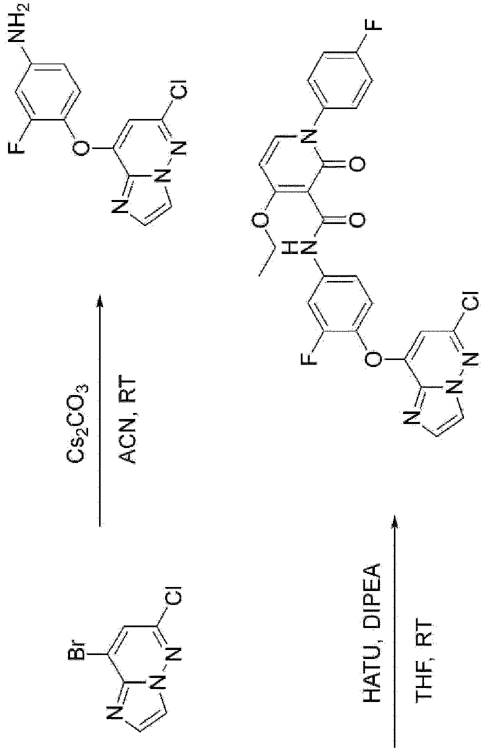

### Step 1) 4-((6-chloroimidazo[1,2-b]pyridazin-8-yl)oxy)-3-fluoroaniline

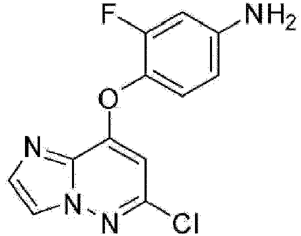

8-bromo-6-chloroimidazo[1,2-b]pyridazine (600 mg, 2.58 mmol) was dissolved in 12 mL of acetonitrile, and Cs₂CO₃ (1.0 g, 3.10 mmol) and 4-amino-2-fluorophenol (361 mg, 2.84 mmol) were added thereto, followed by stirring at room temperature for 12 hours. The reaction mixture was diluted with water and extracted three times with ethyl acetate. The organic layer was washed with brine and concentrated. The residue was purified by silica gel column chromatography to obtain the title compound (435 mg, yield: 61%).

MS m/z : 279[M+H]

### Step 2) N-(4-((6-chloroimidazo[1,2-b]pyridazin-8-yl)oxy)-3-fluorophenyl)-4-ethoxy-1-(4-fluorophenyl)-2-oxo-1,2-dihydropyridine-3-carboxamide

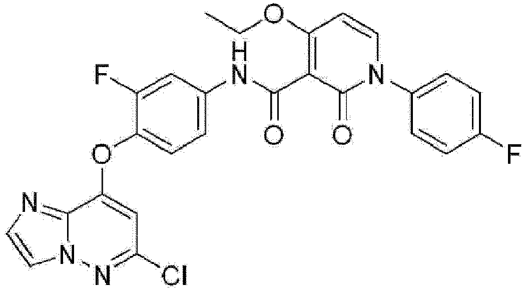

The title compound (210 mg, yield: 54%) was obtained in a manner similar to that of Example 1 using the compound 4-((6-chloroimidazo[1,2-b]pyridazin-8-yl)oxy)-3-fluoroaniline (200 mg, 0.72 mmol) obtained in Step 1 above and 4-ethoxy-1-(4-fluorophenyl)-2-oxo-1,2-dihydropyridine-3-carboxylic acid (240 mg, 0.86 mmol).

¹H NMR (400 MHz, CDCl₃) δ 11.73 (s, 1H), 8.03 (m, 1H), 7.94 (s, 1H), 7.58 (s, 1H), 7.40 (d, 1H), 7.38 (m, 3H), 7.28 - 7.20 (m, 4H), 6.40 (d, 1H), 4.41 (q, 2H), 1.62(t, 3H); MS m/z : 538[M+H]

### Example 6. N-(4-((6-chloroimidazo[1,2-b]pyridazin-8-yl)oxy)-3-fluorophenyl)-1-(4-fluorophenyl)-6-methyl-2-oxo-1,2-dihydropyridine-3-carboxamide

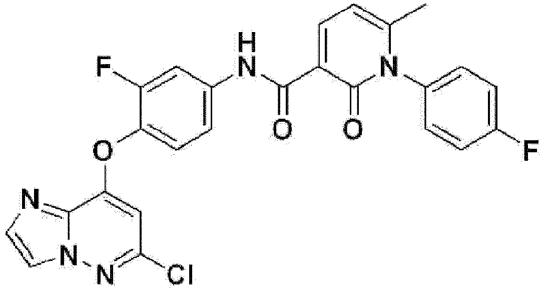

The title compound (21 mg, yield: 57%) was obtained in a manner similar to that of Example 5 using 1-(4-fluorophenyl)-6-methyl-2-oxo-1,2-dihydropyridine-3-carboxylic acid instead of 4-ethoxy-1-(4-fluorophenyl)-2-oxo-1,2-dihydropyridine-3-carboxylic acid according to Step 2 of Example 5.

¹H NMR (400 MHz, CDCl₃) δ 11.99 (s, 1H), 8.66 (m, 1H), 8.04 (d, 1H), 7.94 (s, 1H), 7.76 (s, 1H), 7.37 (m, 3H), 7.28 (m, 3H), 6.54 (d, 1H), 6.18 (s, 1H), 2.15(s, 3H); MS m/z : 508 [M+H]

### Example 7. N-(3-(6-aminopyridin-3-yl)-4-methylphenyl)-4-ethoxy-1-(4-fluorophenyl)-2-oxo-1,2-dihydropyridine-3-carboxamide

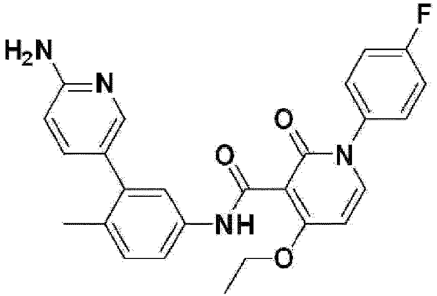

Hereinafter, a compound of Example 7 was prepared according to Reaction Scheme 5 below.

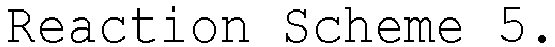

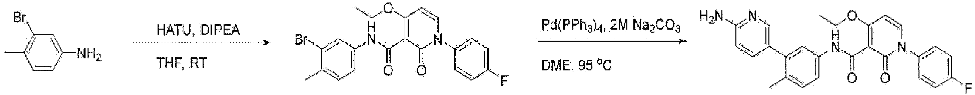

### Step 1) N-(3-bromo-4-methylphenyl)-4-ethoxy-1-(4-fluorophenyl)-2-oxo-1,2-dihydropyridine-3-carboxamide

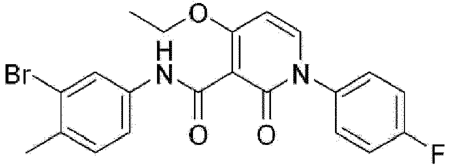

The title compound (700 mg, yield: 59%) was obtained in a manner similar to that of Example 1 by dissolving 3-bromo-4-methylaniline (500 mg, 2.69 mmol) in 10 mL of THF and using 4-ethoxy-1-(4-fluorophenyl)-2-oxo-1,2-dihydropyridine-3-carboxylic acid (894 mg, 3.22 mmol).

MS m/z : 445[M], 447[M+2]

### Step 2) N-(3-(6-aminopyridin-3-yl)-4-methylphenyl)-4-ethoxy-1-(4-fluorophenyl)-2-oxo-1,2-dihydropyridine-3-carboxamide

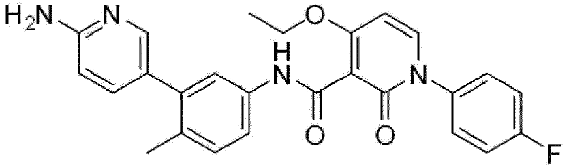

The compound N-(3-bromo-4-methylphenyl)-4-ethoxy-1-(4-fluorophenyl)-2-oxo-1,2-dihydropyridine-3-carboxamide (25 mg, 0.056 mmol) obtained in Step 1 above was dissolved in 2 mL of DME, and (6-aminopyridine-3-yl)boronic acid (9 mg, 0.062 mmol), 2M Na₂CO₃, and Pd(PPh₃)₄ were added thereto, followed by stirring at 95 °C for 12 hours. The reaction mixture was diluted with water, extracted three times with DCM, and washed with brine and concentrated. The residue was purified by silica gel column chromatography to obtain the title compound (8 mg, yield: 30%).

¹H NMR (400 MHz, MeOH-d₄) δ 7.94 (s, 1H), 7.82 (m, 1H), 7.70 (d, 1H), 7.55 (m, 2H), 7.46 (m, 2H), 7.29 (m, 3H), 6.69 (d, 1H), 6.59 (d, 1H), 4.33 (q, 2H), 2.26 (s, 3H), 1.45(t, 3H); MS m/z : 459[M+H]

### Example 8. N-(3-(2-aminopyrimidin-5-yl)-4-methylphenyl)-4-ethoxy-1-(4-fluorophenyl)-2-oxo-1,2-dihydropyridine-3-carboxamide

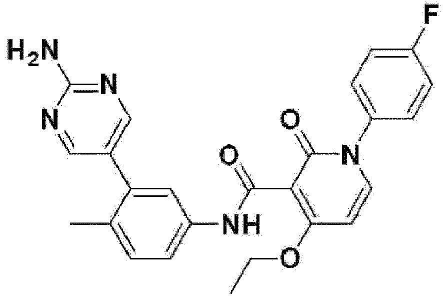

The title compound (12 mg, yield: 32%) was obtained in a manner similar to that of Example 7 using (2-aminopyrimidin-5-yl)boronic acid instead of (6-aminopyridin-3-yl)boronic acid according to Step 2 of Example 7.

¹H NMR (400 MHz, MeOH-d₄) δ 8.30 (s, 2H), 7.76 (m, 1H), 7.57 (m, 2H), 7.44 (m, 2H), 7.29 (m, 3H), 6.59 (d, 1H), 4.31 (q, 2H), 2.28 (s, 3H), 1.44(t, 3H); MS m/z : 460[M+H]

### Example 9. N-(3-(2-aminopyridin-4-yl)-4-methylphenyl)-4-ethoxy-1-(4-fluorophenyl)-2-oxo-1,2-dihydropyridine-3-carboxamide

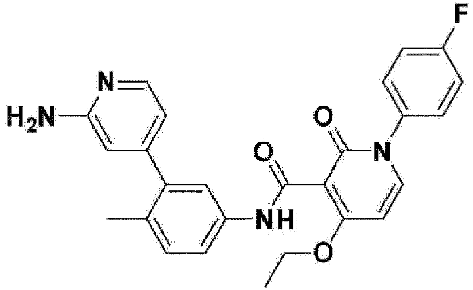

The title compound (2 mg, yield: 5%) was obtained in a manner similar to that of Example 7 using 4-(4,4,5,5-tetramethyl-1,3,2-dioxoborolan-2-yl)pyridine-2-amine instead of (6-aminopyridin-3-yl)boronic acid according to Step 2 of Example 7.

¹H NMR (400 MHz, MeOH-d₄) δ 7.92 (brs, 1H), 7.78 (d, 1H), 7.59 (m, 2H), 7.45 (m, 2H), 7.28 (m, 3H), 6.60 (m, 3H), 4.34 (q, 2H), 2.26 (s, 3H), 1.45(t, 3H); MS m/z : 459[M+H]

### Example 10. N-(3-(2-aminopyridin-3-yl)-4-methylphenyl)-4-ethoxy-1-(4-fluorophenyl)-2-oxo-1,2-dihydropyridine-3-carboxamide

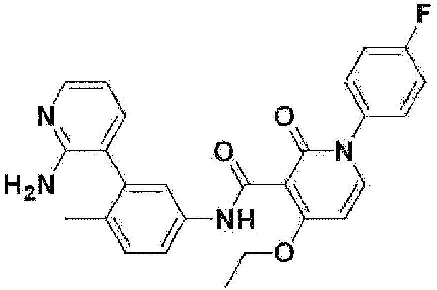

The title compound (12 mg, yield: 38%) was obtained in a manner similar to that of Example 7 using 3-(4,4,5,5-tetramethyl-1,3,2-dioxoborolan-2-yl)pyridine-2-amine instead of (6-aminopyridin-3-yl)boronic acid according to Step 2 of Example 7.

¹H NMR (400 MHz, MeOH-d₄) δ 7.90 (brs, 2H), 7.77 (d, 1H), 7.62 (d, 1H), 7.50 (m, 2H), 7.45 (m, 2H), 7.35 (m, 4H), 6.77 (m, 1H), 6.59 (d, 1H), 4.33 (q, 2H), 2.14 (s, 3H), 1.44(t, 3H); MS m/z : 459[M+H]

### Example 11. 4-ethoxy-1-(4-fluorophenyl)-N-(4-methyl-3-(quinolin-6-yl)phenyl)-2-oxo-1,2-dihydropyridine-3-carboxamide

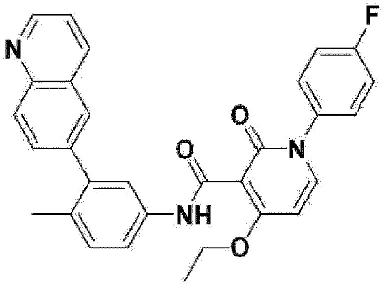

The title compound (13 mg, yield: 40%) was obtained in a manner similar to that of Example 7 using quinolin-6-yl-boronic acid instead of (6-aminopyridin-3-yl)boronic acid according to Step 2 of Example 7.

¹H NMR (400 MHz, MeOH-d₄) δ 8.89 (brs, 1H), 8.42 (m, 1H), 8.09 (m, 1H), 7.91 (s, 1H), 7.81 (m, 2H), 7.70 (m, 1H), 7.60 (t, 2H), 7.45 (m, 2H), 7.30 (m, 3H), 6.58 (d, 1H), 4.33 (q, 2H), 2.28 (s, 3H), 1.44(t, 3H); MS m/z : 494 [M+H]

### Example 12. 4-ethoxy-1-(4-fluorophenyl)-N-(3-(isoquinolin-6-yl)-4-methylphenyl)-2-oxo -1,2-dihydropyridine-3-carboxamide

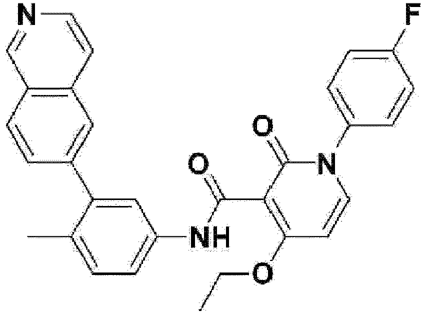

The title compound (9 mg, yield: 27%) was obtained in a manner similar to that of Example 7 using isoquinolin-6-yl-boronic acid instead of (6-aminopyridin-3-yl)boronic acid according to Step 2 of Example 7.

¹H NMR (400 MHz, MeOH-d₄) δ 9.30 (s, 1H), 8.50 (m, 1H), 8.18 (d, 1H), 7.87 (m, 2H), 7.73 (m, 2H), 7.70 (m, 1H), 7.60 (m, 1H), 7.45 (m, 2H), 7.36 (m, 3H), 6.59 (d, 1H), 4.33 (q, 2H), 2.27 (s, 3H), 1.45(t, 3H); MS m/z : 494[M+H]

### Example 13. 4-ethoxy-1-(4-fluorophenyl)-N-(4-methyl-3-(1H-pyrrolo[2,3-b]pyridin-4-yl)phenyl)-2-oxo-1,2-dihydropyridine-3-carboxamide

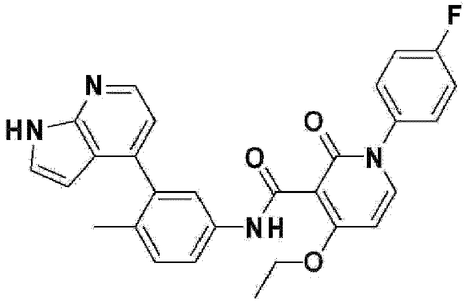

The title compound (6 mg, yield: 18%) was obtained in a manner similar to that of Example 7 using 4-(4,4,5,5-tetramethyl-1,3,2-dioxoborolan-2-yl)-1H-pyrrolo[2,3-b]pyridine instead of (6-aminopyridin-3-yl)boronic acid according to Step 2 of Example 7.

¹H NMR (400 MHz, MeOH-d₄) δ 8.24 (d, 1H), 7.91 (s, 1H), 7.72 (m, 1H), 7.67 (m, 2H), 7.45 (m, 3H), 7.34 (d, 1H), 7.29 (m, 2H), 7.04 (d, 1H), 6.58 (d, 1H), 6.28 (d, 1H), 4.32 (q, 2H), 2.17 (s, 3H), 1.44(t, 3H);

MS m/z : 483[M+H]

### Example 14. N-(4-((3-bromoimidazo[1,2-b]pyridazin-6-yl)oxy)-2-fluorophenyl)-4-ethoxy-1-(4-fluorophenyl)-2-oxo-1,2-dihydropyridine-3-carboxamide

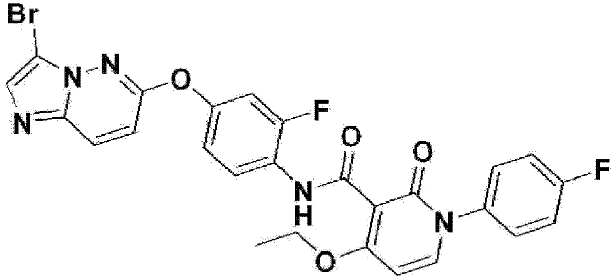

The title compound (10 mg, yield: 39.0%) was obtained in a manner similar to that of Example 5 using 4-(3-bromoimidazo[1,2-b]pyridazin-6-yl)oxy)-2-fluoroaniline instead of compound 4-(6-chloroimidazo[1,2-b]pyridazin-8-yl)-3-fluoroaniline according to Step 2 of Example 5.

MS m/z : 582[M], 584[M+2]

### Example 15. N-(4-(2-(2-aminopyrimidin-4-yl)pyridin-4-yl)-3-fluorophenyl)-1-(4-fluorophenyl)-4-methoxy-2-oxo-1,2-dihydropyridine-3-carboxamide

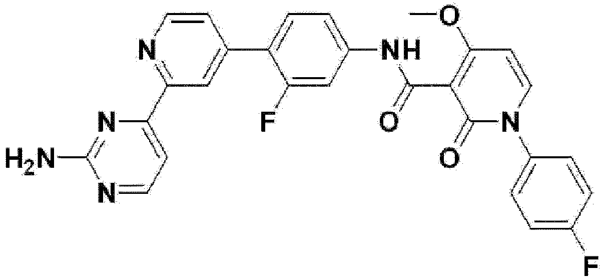

Hereinafter, a compound of Example 15 was prepared according to Reaction Scheme 6 below.

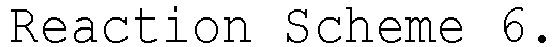

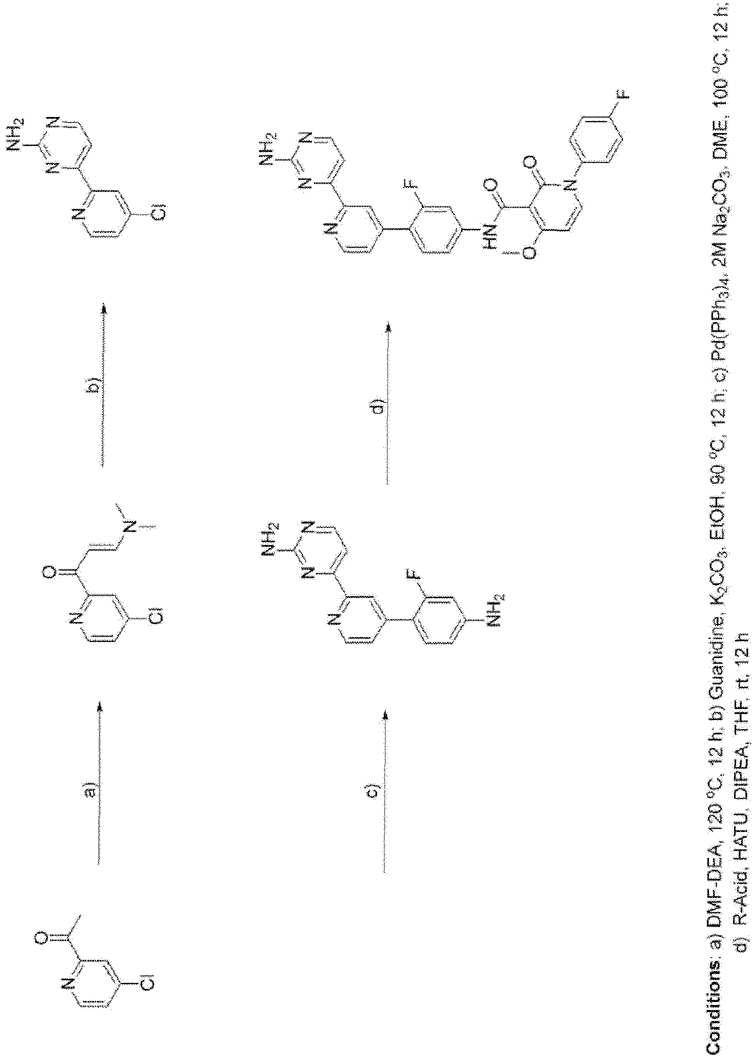

### Step 1. (E)-1-(4-chloropyridine-2-yl)-3-(dimethylamino)prop-2-en-1-one

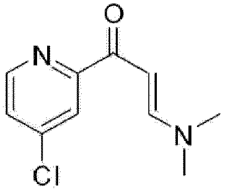

1-(4-chloropyridine-2-yl)ethane-1-one (200 mg, 1.29 mmol) and N,N'-dimethylformamide diethylacetal (1.5 mL, 9.0 mmol) were mixed and stirred under reflux at 120 °C for 12 hours. The reaction was completed, and then the reaction solution was concentrated to obtain the title compound (250 mg, yield: 93%).

MS *m*/*z* : 211[M+H]

### Step 2. 4-(4-chloropyridin-2-yl)pyrimidine-2-amine

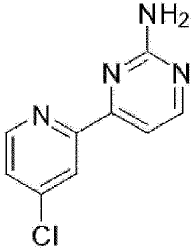

The compound ((E)-1-(4-chloropyridine-2-yl)-3-(dimethylamino)prop-2-en-1-one (250 mg, 1.18 mmol) obtained in Step 1 above was dissolved in 7 mL of ethanol, and then guanidine hydrochloride (147 mg, 1.54 mmol) and K₂CO₃ (355 mg, 2.57 mmol) were added thereto, followed by stirring under reflux for 12 hours. The reaction was completed, and then the reaction solution was diluted with water, and extracted three times with DCM and concentrated. The residue was purified by silica gel column chromatography to obtain the title compound (170 mg, yield: 70%).

MS m/z : 207[M+H]

### Step 3. 4-(4-(4-amino-2-fluorophenyl)pyridin-2-yl)pyrimidine-2-amine

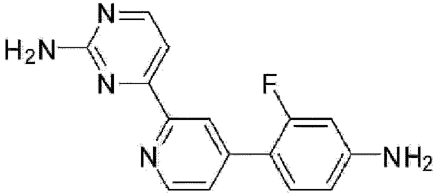

The compound 4-(4-chloropyridin-2-yl)pyrimidine-2-amine (90 mg, 0.43 mmol) obtained in Step 2 above was dissolved in 3 mL of DME, and then 3-fluoro-4-(4,4,5,5-tetramethyl-1,3,2-dioxaborolan-2-yl)aniline (124 mg, 0.52 mmol), 2 N Na2CO₃ aqueous solution (0.54 mL, 1.09 mmol), and Pd(PPh₃)₄ (10 mg, 0.01 mmol) were added thereto, followed by stirring at 95 °C for 12 hours. The solution was cooled, diluted with water, and then extracted three times with DCM and concentrated. The residue was purified by silica gel column chromatography to obtain the title compound (100 mg, yield: 82%).

MS *m*/*z* : 281[M+H]

### Step 4. N-(4-(2-(2-aminopyrimidin-4-yl)pyridin-4-yl)-3-fluorophenyl)-1-(4-fluorophenyl)-4-methoxy-2-oxo-1,2-dihydropyridine-3-carboxamide

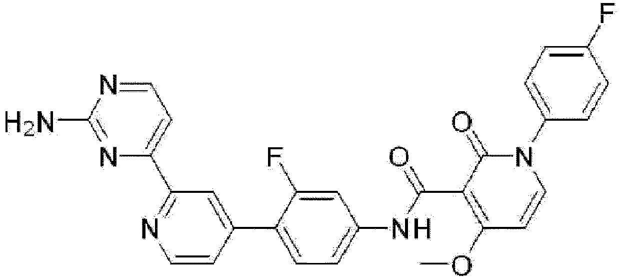

The compound 4-(4-(4-amino-2-fluorophneyl)pyridin-2-yl)pyrimidine-2-amine (30 mg, 0.11 mmol) obtained in Step 3 above was dissolved in 3 mL of THF, and then 1-(4-fluorophenyl) -4-methoxy-2-oxo-1,2-dihydropyridine-3-carboxylic acid (34 mg, 0.13 mmol), HATU (61 mg, 0.07 mmol), and DIPEA (37 µL, 0.21 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (21 mg, 37%).

¹H NMR (400 MHz, DMSO-d₆) δ 11.76 (s, 1H), 8.78 (d, 1H), 8.52 (d, 1H), 8.43 (m, 1), 7.91 (m, 2H), 7.71 (m, 2H), 7.55 (m, 2H), 7.46 (m, 2H), 7.37 (m, 2H), 6.87 (brs, 2H), 6.56 (d, 1H), 3.93 (s, 3H); MS m/z : 527[M+H]

### Example 16. N-(4-(2-(2-aminopyrimidin-4-yl)pyridin-4-yl)-3-fluorophenyl)-1-(4-fluorophenyl)-6-methyl-2-oxo-1,2-dihydropyridine-3-carboxamide

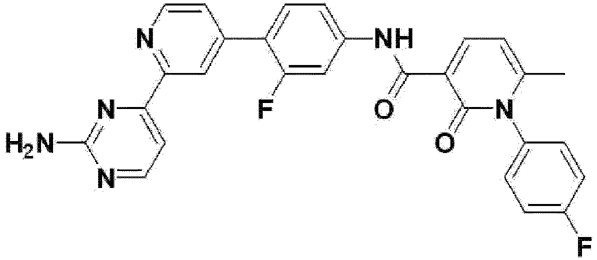

The title compound (6 mg, yield: 11%) was obtained in a manner similar to that of Example 15 using 1-(4-fluorophenyl)-6-methyl-2-oxo-1,2-dihydropyridine-3-carboxylic acid instead of 1-(4-fluorophenyl)-4-methoxy-2-oxo-1,2-dihydropyridine-3-carboxylic acid according to Step 4 of Example 15.

¹H NMR (400 MHz, DMSO-d₆) δ 12.18 (s, 1H), 8.77 (d, 1H), 8.52 (m, 2H), 8.42 (m, 1), 7.99 (m, 1H), 7.72 (m, 2H), 7.57 - 7.43(m, 6H), 6.78 (brs, 2H), 6.74 (d, 1H), 2.09 (s, 3H); MS *m*/*z* : 511[M+H]

### Example 17. N-(4-(2-(2-aminopyrimidin-4-yl)pyridin-4-yl)-3-fluorophenyl)-5-bromo-1-(4-fluorophenyl)-2-oxo-1,2-dihydropyridine-3-carboxamide

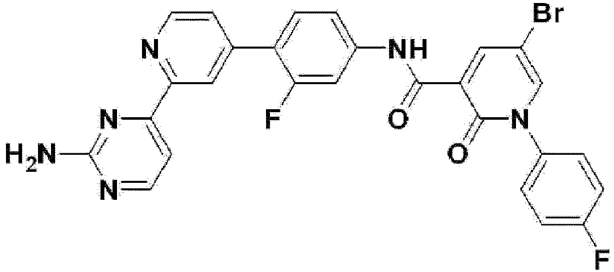

The title compound (6 mg, yield: 7%) was obtained in a manner similar to that of Example 15 using 5-bromo-1-(4-fluorophenyl)-2-oxo-1,2-dihydropyridine-3-carboxylic acid instead of 1-(4-fluorophenyl)-4-methoxy-2-oxo-1,2-dihydropyridine-3-carboxylic acid according to Step 4 of Example 15.

¹H NMR (400 MHz, DMSO-d₆) δ 12.08 (s, 1H), 8.89 (s, 1H), 8.56 (m, 3H), 8.40 (m, 1), 8.02 (m, 1H), 7.74 (m, 2H), 7.56 (m, 3H), 7.45 (m, 1H), 7.38 (m, 2H), 6.79 (brs, 2H);

MS m/z : 575[M], 577[M+2]

### Example 18. N-(4-(3-amino-1H-indazol-6-yl)-3-fluorophenyl)-4-ethoxy-1-(4-fluorophenyl)-2-oxo-1,2-dihydropyridine-3-carboxamide

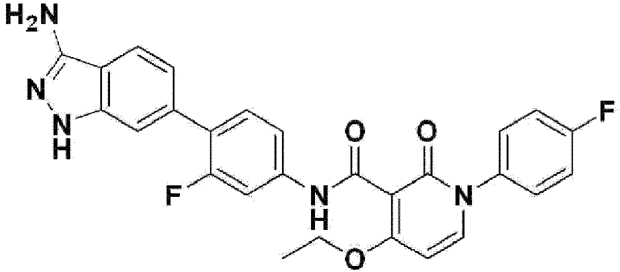

Hereinafter, a compound of Example 18 was prepared according to Reaction Scheme 7 below.

Reaction Scheme 7.

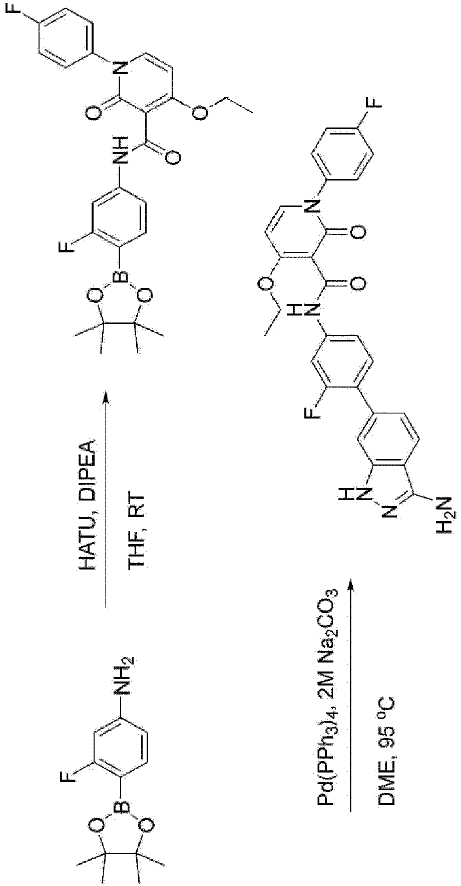

### Step 1. 4-ethoxy-N-(3-fluoro-4-(4,4,5,5-tetramethyl-1,3,2-dioxaborolan-2-yl)phenyl-1-(4-fluorophenyl)-2-oxo-1,2-dihydropyridine-3-carboxamide

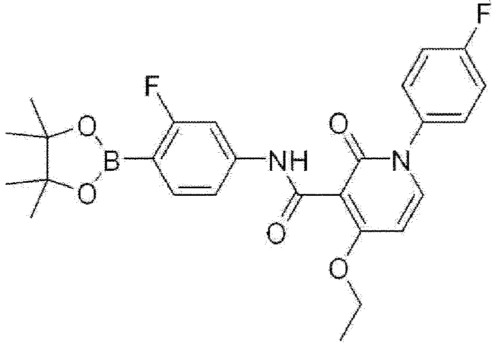

3-fluoro-4-(4,4,5,5-tetramethyl-1,3,2-dioxaborolan-2-yl)aniline (500 mg, 2.11 mmol) was dissolved in 10 mL of THF, and then 4-ethoxy-1-(4-fluorophenyl)-2-oxo-1,2-dihydropyridine-3-carboxylic acid (702 mg, 2.53 mmol), HATU (1200 mg, 3.16 mmol), and DIPEA (553 µL, 3.16 mmol) were added thereto, followed by stirring at room temperature for 12 hours. The reaction solution was diluted with water, and then extracted three times with ethyl acetate and concentrated. The residue was purified by silica gel column chromatography to obtain the title compound (450 mg, 43%).

MS *m*/*z* : 497[M+H]

### Step 2. N-(4-(3-amino-1H-indazol-6-yl)-3-fluorophenyl)-4-ethoxy-1-(4-fluorophenyl)-2-oxo-1,2-dihydropyridine-3-carboxamide

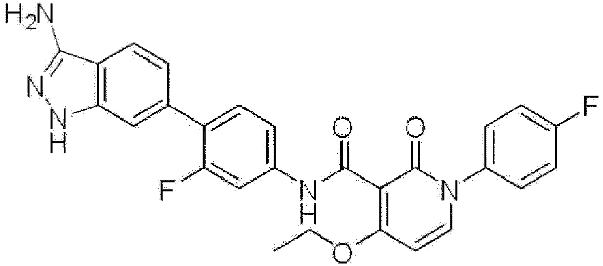

The compound 4-ethoxy-N-(3-fluoro-4-(4,4,5,5-tetramethyl-1,3,2-dioxaborolan-2-yl)phenyl-1-(4-fluorophenyl)-2-oxo-1,2-dihydropyridine-3-carboxamide (77 mg, 0.16 mmol) obtained in Step 1 above was dissolved in 2 mL of 1,4-dioxane, and then 6-bromo-1H-indazole-3-amine (30 mg, 0.14 mmol), a saturated Na₂CO₃ aqueous solution (0.14 ml, 0.28 mmol), PdCl₂(dppf)DCM adduct (12 mg, 0.014 mmol) was added thereto, followed by stirring at 90 °C for 12 hours. The reaction mixture was filtered through celite, and the filtrate was dried under vacuum. The residue was purified by prep-HPLC (0.1% formic acid in water/acetonitrile) to obtain the title compound (17 mg, yield: 24%).

¹H NMR (400 MHz, DMSO-d₆) δ 11.43 (s, 1H), 10.53 (s, 1H), 7.87 (m, 1H), 7.79 (m, 2H), 7.55 (m, 4H), 7.39 (m, 3H), 7.07 (m, 1H), 6.53 (d, 1H), 5.37 (brs, 2H), 4.28 (q, 2H), 1.32(t, 3H);

MS m/z : 502[M+H]

### Example 19. 4-ethoxy-N-(3-fluoro-4-(1H-indazole-5-yl)phenyl)-1-4-fluorophenyl)-2-oxo-1,2-dihydropyridine-3-carboxamide

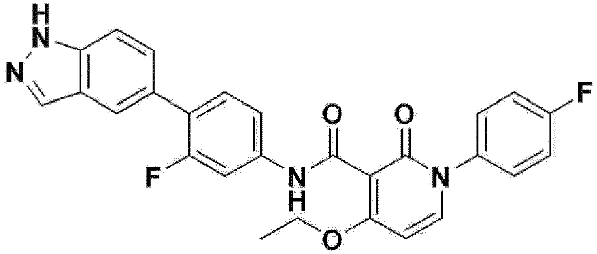

The title compound (17 mg, yield: 23%) was obtained in a manner similar to that of Example 18 using 5-bromo-1H-indazole instead of 6-bromo-1H-indazole-3-amine according to Step 2 of Example 18.

¹H NMR (400 MHz, DMSO-d₆) δ 10.52 (s, 1H), 8.13 (s, 1H), 7.91 (d, 1H), 7.85 (d, 1H), 7.76 (m, 1H), 7.54 (m, 1H), 7.49 (m, 5H), 7.36 (m, 2H), 6.52 (d, 1H), 4.28 (q, 2H), 1.32(t, 3H); MS m/z : 487[M+H]

### Example 20. 4-ethoxy-N-(3-fluoro-4-(2-oxoindolin-5-yl)phenyl)-1-(4-fluorophenyl)-2-oxo-1,2-dihydropyridine-3-carboxamide

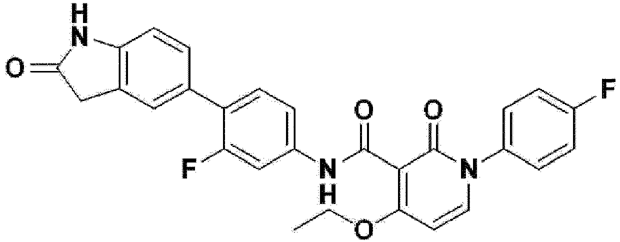

The title compound (20 mg, yield: 29%) was obtained in a manner similar to that of Example 18 using 5-bromoindoline-2-one instead of 6-bromo-1H-indazole-3-amine according to Step 2 of Example 18.

¹H NMR (400 MHz, DMSO-d₆) δ 10.49 (d, 1H), 10.46 (d, 1H), 7.86 (d, 1H), 7.75 (d, 1H), 7.48 (m, 4H), 7.39 (m, 4H), 6.91(d, 1H), 6.52 (d, 1H), 4.27 (q, 2H), 3.54 (s, 2H), 1.31(t, 3H); MS m/z : 502[M+H]

### Example 21. N-(4-(3-aminobenzo[d]isothiazol-5-yl)-3-fluorophenyl)-4-ethoxy-1-(4-fluorophenyl)-2-oxo-1,2-dihydropyridine-3-carboxamide

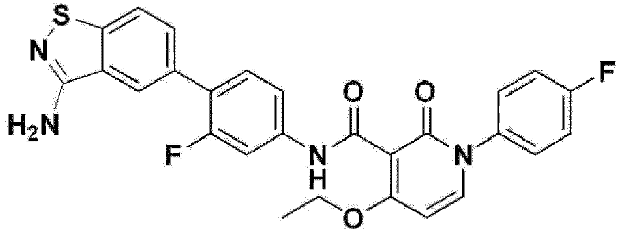

The title compound (36 mg, yield: 52%) was obtained in a manner similar to that of Example 18 using 5-bromobenzo[d]isothiazole-3-amine instead of 6-bromo-1H-indazole-3-amine according to Step 2 of Example 18.

¹H NMR (400 MHz, DMSO-d₆) δ 10.57 (s, 1H), 8.29 (s, 1H), 8.01 (m, 1H), 7.87 (d, 1H), 7.65 (m, 1H), 7.47 (m, 4H), 7.39 (m, 2H), 6.86 (brs, 2H), 6.53 (d, 1H), 4.29 (q, 2H), 1.32(t, 3H); MS m/z : 519[M+H]

### Example 22. N-(4-(3-amino-1H-indazol-6-yl)-3-fluorophenyl)-1-(2-hydroxy-2-methylpropyl)-5-methyl-3-oxo-2-phenyl-2,3-dihydro-1H-pyrazole-4-carboxamide

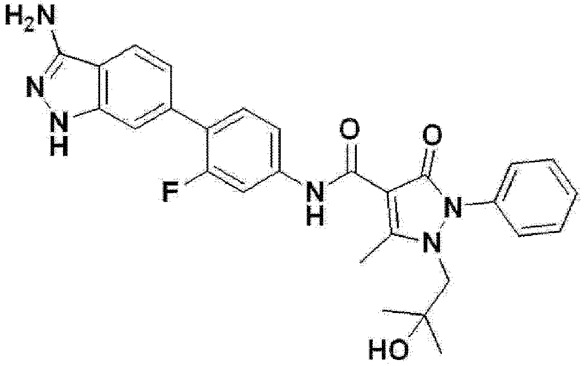

Hereinafter, a compound of Example 22 was prepared according to Reaction Scheme 8 below. 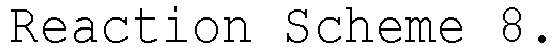 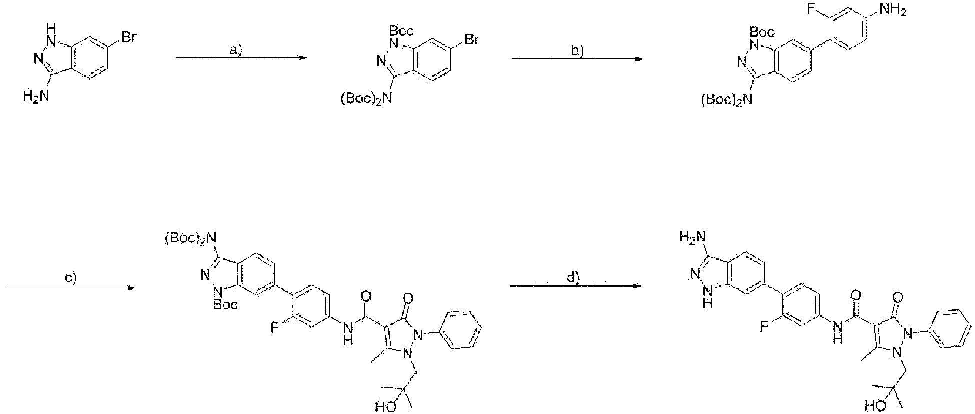 Conditions: a) Boc-anhydride, DMAP, THF, rt, 12 h; b) Pd(PPh₃)₄, 2M Na₂CO₃, DME, 100 °C, 12 h; c) R-Acid, HATU, DIPEA, THF, rt, 12 h; d) DCM, TFA, rt, 2 h

### Step 1. Boc-protected 6-bromoindazoleamine

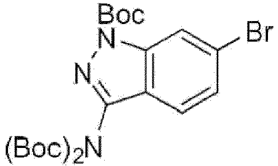

6-bromo-1H-indazole-3-amine (500 mg, 2.36 mmol) was dissolved in 15 mL of acetonitrile, Boc-anhydride (2.74 ml, 11.8 mmol) and DMAP (14 mg, 0.12 mmol) were added thereto, followed by stirring at 90 °C for 3 hours. The reaction was completed, and then the reaction solution was diluted with water, and extracted three times with EA and concentrated. The residue was purified by silica gel column chromatography to obtain the title compound (900 mg, yield: 75%).

MS m/z : 512[M], 514[M+2]

### Step 2. T-butyl-3-((di-t-buthoxycarbonyl)amino)-1H-indazole-1-carboxylate

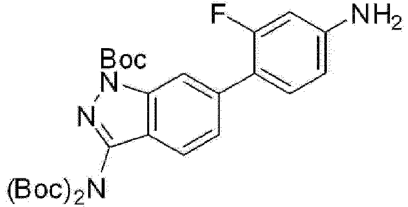

The compound Boc-protected 6-bromoindazoleamine (900 mg, 1.76 mmol) obtained in Step 1 above was dissolved in 10 mL of DME, and then 3-fluoro-4-(4,4,5,5-tetramethyl-1,3,2-dioxaborolan-2-yl)aniline (500 mg, 2.11 mmol), 2 N Na₂CO₃ aqueous solution (2.2 mL, 4.39 mmol), and Pd(PPh₃)₄ (41 mg, 0.035 mmol) were added thereto, followed by stirring at 95 °C for 12 hours. The reaction mixture was diluted with water and extracted three times with DCM. The organic layer was washed with brine and concentrated. The residue was purified by silica gel column chromatography to obtain the title compound (900 mg, yield: 94%).

MS m/z : 543[M+H]

### Step 3. T-butyl-3-((di-t-butoxycarbonyl)amino)-6-(2-fluoro-4-(1-phenyl-5-methyl-3-oxo-2-phenyl-2,3-dihydro-1H-pyrazol-4-carboxamido)-indazole-1-carboxylate

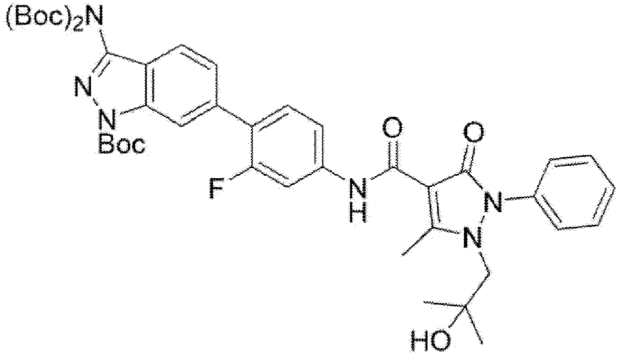

The compound t-butyl t-butyl-3-((di-t-buthoxycarbonyl)amino)-1H-indazole-1-carboxylate (90 mg, 0.16 mmol) obtained in Step 2 above was dissolved in 2 mL of THF, and then 1-(2-hydroxy-2-methylpropyl)-5-methyl-3-oxo-2-phenyl-2,3-dihydro-1H-pyrrazole-4-carboxylic acid (51 mg, 0.20 mmol), HATU (95 mg, 0.25 mmol), and DIPEA (43 µL, 0.25 mmol) were added thereto, followed by stirring at room temperature for 12 hours. The reaction solution was diluted with water, and then extracted three times with EA and concentrated. The residue was purified by silica gel column chromatography to obtain the title compound (90 mg, 70%).

MS m/z : 815[M+H]

### Step 4. N-(4-(3-amino-1H-indazol-6-yl)-3-fluorophenyl)-1-(2-hydroxy-2-methylpropyl)-5-methyl-3-oxo-2-phenyl-2,3-dihydro-1H-pyrazole-4-carboxamide

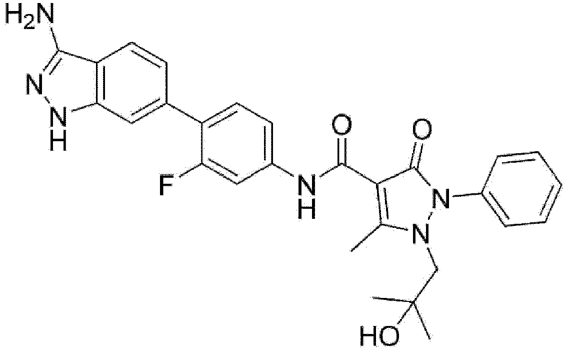

The compound t-butyl-3-((di-t-butoxycarbonyl)amino)-6-(2-fluoro-4-(1-phenyl-5-methyl-3-oxo-2-phenyl-2,3-dihydro-1H-pyrazole-4-carboxamido)-indazole-1-carboxylate (90 mg, 0.11 mmol) obtained in Step 3 was dissolved in 1.5 mL of DCM, 1.5 mL of TFA was added thereto, followed by stirring at room temperature for 2 hours. The reaction solution was dried under vacuum. The residue was dissolved in DCM, then neutralized with a saturated NaHCO₃ aqueous solution, and extracted three times with DCM and concentrated. The residue was purified by prep-HPLC (0.1% formic acid in water/acetonitrile) to obtain the title compound (31 mg, yield: 55%).

¹H NMR (400 MHz, DMSO-d₆) δ 11.46 (s, 1H), 10.87 (s, 1H), 7.82 (m, 1H), 7.74 (m, 1H), 7.59 (m, 2H), 7.45 (m, 2H), 7.33 (m, 3H), 7.28 (m, 1H), 7.02 (m, 1H), 5.36 (brs, 2H), 4.80 (s, 1H), 3.84 (brs, 2H), 2.79 (s, 3H), 0.98(s, 6H); MS m/z : 515[M+H]

### Example 23. N-(4-(3-amino-1H-indazol-6-yl)-3-fluorophenyl)-1-(4-fluorophenyl)-6-methyl-2-oxo-1,2-dihydropyridine-3-carboxamide

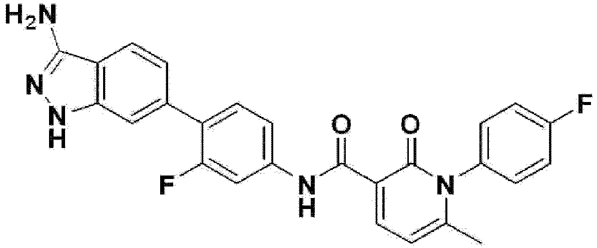

The title compound (30 mg, yield: 55%) was obtained in a manner similar to that of Example 22 using 1-(4-fluorophenyl)-6-methyl-2-oxo-1,2-dihydropyridine-3-carboxylic acid instead of 1-phenyl-5-(trifluoromethyl)-1H-imidazole-4-carboxylic acid according to Step 3 of Example 22.

¹H NMR (400 MHz, DMSO-d₆) δ 12.07 (s, 1H), 11.45 (s, 1H), 8.52 (d, 1H), 7.91 (d, 1H), 7.75 (d, 1H), 7.53 (m, 3H), 7.44 (m, 3H), 7.35 (s, 1H), 7.07 (d, 1H), 6.73 (d, 1H), 5.37 (brs, 2H), 2.09(s, 3H); MS m/z : 472[M+H]

### Example 24. N-(4-((2-amino-3-chloropyridin-4-yl)oxy)-3-fluorophenyl)-1,5-dimethyl-3-oxo-2-phenyl-2,3-dihydro-1H-pyrazole-4-carboxamide

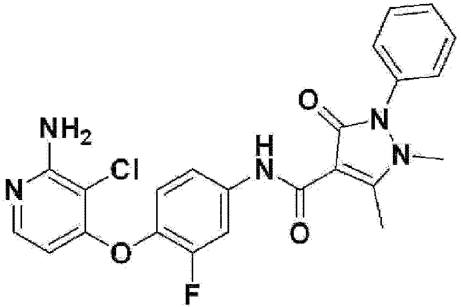

Hereinafter, a compound of Example 24 was prepared according to Reaction Scheme 9 below. 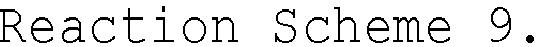 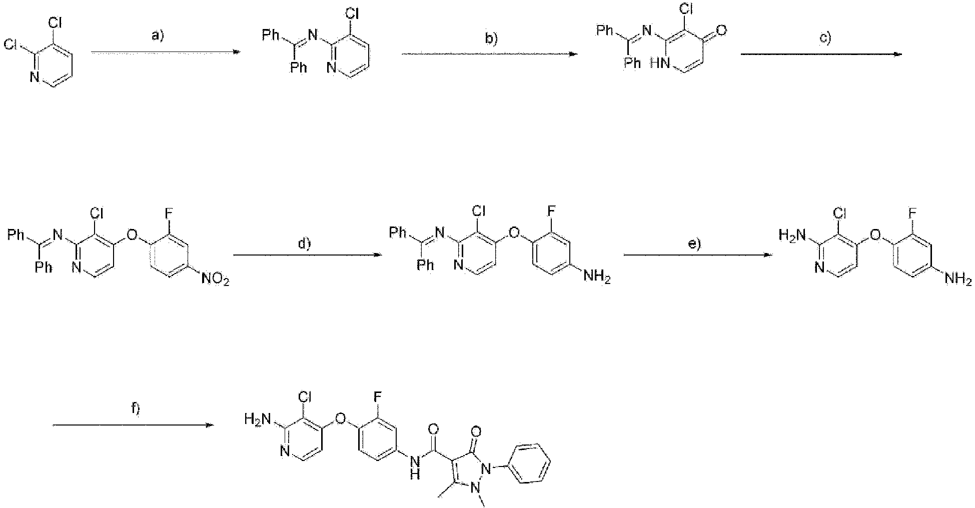 Conditions: a) Pd(OAc)₂, Cs₂CO₃, BINAP, MTBE, 70 °C; b) LDA, B(iPrO)₃, THF, NaHSO₃, H₂O; c) 3,4-Difluoronitrobenzne, Cs₂CO₃, DMF, d) (NH₄)₂S, i-PrOH, 70 °C; e) 1M HCl, THF; f) Acid, HATU, DIPEA, THF

### Step 1) N-(3-chloropyridin-2-yl)-1,1-diphenylmethanimine

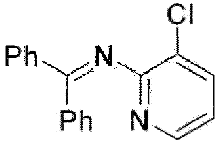

2,3-dichloropyridine (21 g, 142 mmol) was dissolved in methyl-tert-butyl ether (MTBE), and then Pd(OAc)2 (318 mg, 1.42 mmol), rac-BINAP (1.4 g, 2.13 mmol), Cs2CO3 (0.9 g, 2.84 mmol), and benzophenone imine (26 g, 142 mmol) were added thereto. The reaction mixture was stirred under reflux at 70 °C for 12 hours. The reaction mixture was cooled to room temperature, and the resulting solids were filtered with celite to concentrate the solvent. The residue was purified by column chromatography to obtain the title compound (20 g, yield: 48%) .

MS m/z : 293 [M+H]

### Step 2) N-(3-chloropyridin-2-yl)-1,1-diphenylmethanimine

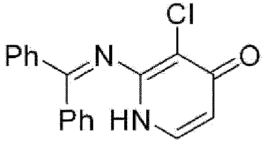

The compound N-(3-chloropyridin-2-yl)-1,1-diphenylmethanimine (20 g, 68.3 mmol) obtained in Step 1 above was dissolved in THF 100 and tri-isopropyl borate (19.3 g, 102 mmol) was added thereto, followed by cooling to 0 °C. To the reaction mixture, lithium diisopropylamide (11 mL, 88.8 mmol) was slowly added at 0 °C. The reaction mixture was stirred at 0 °C for 2 hours, water (100 mL) was added thereto, and then sodium percarbonate (16 g, 102 mmol) was added thereto, followed by further stirring at room temperature for 3 hours. 50 mL of a saturated NaHSO₃ was slowly added to the reaction mixture, and extracted three times with EA. The organic layer was concentrated and used in Step 3 below without a purification process.

MS m/z : 309 [M+H]

### Step 3) N-(3-chloro-4-(2-fluoro-4-nitrophenoxy)pyridin-2-yl-1,1-diphenylmethanimine

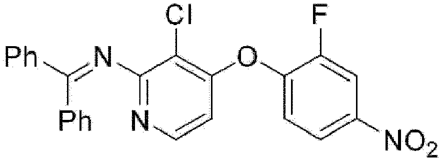

The compound N-(3-chloropyridin-2-yl)-1,1-diphenylmethanimine (20 g, 65 mmol) was dissolved in DMF, and then 3,4-difluoronitrobenzne (9.3 ml, 84.5 mmol) and Cs₂CO₃ (27.5 g, 84.5 mmol) were added thereto, followed by stirring at 90 °C for 1 hour. The reaction mixture was cooled to room temperature, diluted with water, and extracted three times with EA. The organic solution was washed with brine and concentrated. The residue was purified by column chromatography to obtain the title compound (21 g, yield: 72%) .

MS m/z : 448 [M+H]

### Step 4) 4-((3-chloro-2-(diphenylmethylene)amino)pyridin-4-yl)oxy-3-fluoroaniline

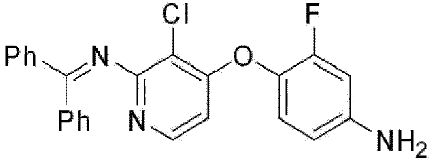

The compound N-(3-chloro-4-(2-fluoro-4-nitrophenoxy)pyridin-2-yl-1,1-diphenylmethanimine (20 g, 44.6 mmol) obtained in Step 3 above was dissolved in isopropanol, ammonium sulfide (30.4 ml, 446 mmol) was added thereto, followed by stirring at room temperature for 1 hour. Thereafter, the reaction mixture was stirred again at 70 °C for 3 hours. After the reaction was completed, water was added thereto, and the reaction mixture was cooled to room temperature. The resulting solids were obtained by filtering, butyl acetate was added thereto, and heated and dissolved at 80 °C, heptane was added thereto, and the reaction mixture was cooled to room temperature. After the reaction mixture was cooled to room temperature, the resulting solids were filtered and dried to obtain the title compound (14 g, 75%).

MS m/z : 418 [M+H]

### Step 5) 4-(4-amino-2-fluorophenoxy)-3-chloropyridin-2-amine

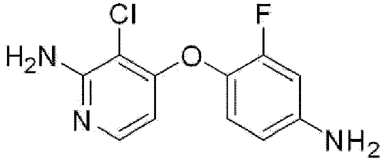

The compound 4-((3-chloro-2-(diphenylmethylene)amino)pyridine-4-yl)oxy-3-fluoroaniline (14 g, 33 mmol) obtained in Step 4 above was dissolved in THF, 1 M HCl (14 ml) was added thereto, followed by stirring at room temperature for 2 hours. The reaction mixture was concentrated, and then saturated NaHCO₃ was added thereto, and the reaction mixture was extracted three times with EA. The organic solution was washed with brine and concentrated. The residue was purified by column chromatography to obtain the title compound (7.1 g, yield: 84%).

MS m/z : 254 [M+H]

### Step 6) N-(4-((2-amino-3-chloropyridin-4-yl)oxy)-3-fluorophenyl)-1,5-dimethyl-3-oxo-2-phenyl-2,3-dihydro-1H-pyrazole-4-carboxamide

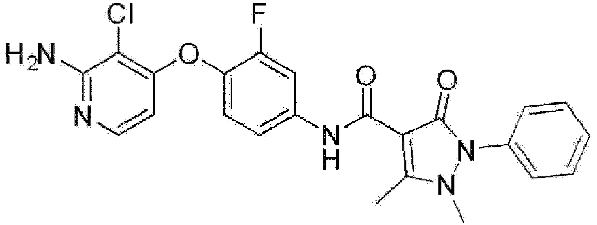

The compound 4-(4-amino-2-fluorophenoxy)-3-chloropyridine-2-amine (70 mg, 0.28 mmol) obtained in Step 5 above was dissolved in 3 mL of THF, and then 1,5-dimethyl-3-oxo-2-phenyl-2,3-dihydro-1H-pyrazole-4-carboxylic acid (77 mg, 0.33 mmol), HATU (156 mg, 0.41 mmol), and DIPEA (70 µL, 0.41 mmol) were added thereto, followed by stirring at room temperature for 12 hours. The reaction solution was diluted with water, and then extracted three times with EA and concentrated. The residue was purified by prep-HPLC (0.1% formic acid in water/acetonitrile) to obtain the title compound (74 mg, 57%).

¹H NMR (400 MHz, DMSO-d₆) δ 10.93 (s, 1H), 7.94 (d, 1H), 7.76 (d, 1H), 7.61 (m, 2H), 7.58 (m, 1H), 7.45 (m, 2H), 7.29 (m, 2H), 6.40 (brs, 2H), 5.94 (d, 1H), 3.37 (s, 3H), 2.71(s, 3H); MS *m*/*z* : 468[M+H]

### Example 25. N-(4-((2-amino-3-chloropyridin-4-yl)oxy)-3-fluorophenyl)-1-(4-fluorophenyl)-6-methyl-2-oxo-1,2-dihydropyridine-3-carboxamide

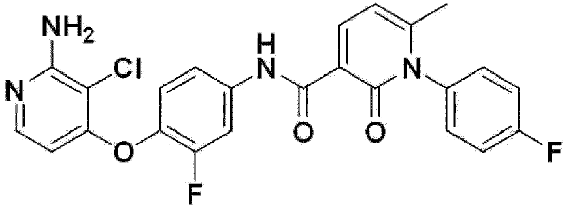

The title compound (39 mg, yield: 75%) was obtained in a manner similar to that of Example 24 using 1-(4-fluorophenyl)-6-methyl-2-oxo-1,2-dihydropyridine-3-carboxylic acid instead of 1,5-dimethyl-3-oxo-2-phenyl-2,3-dihydro-1H-pyrazole-4-carboxylic acid according to Step 6 of Example 24.

¹H NMR (400 MHz, DMSO-d₆) δ 12.05 (s, 1H), 8.51 (d, 1H), 8.00 (m, 1H), 7.76 (d, 1H), 7.49 (m, 5H), 7.29 (t, 1H), 6.73 (d, 1H), 6.40 (brs, 2H), 5.94(d, 1H), 2.09(s, 3H); MS m/z : 483[M+H]

### Example 26. N-(4-((2-amino-3-chloropyridin-4-yl)oxy)-3-fluorophenyl)-1-(4-fluorophenyl)-6-(hydroxymethyl)-2-oxo-1,2-dihydropyridine-3-carboxamide

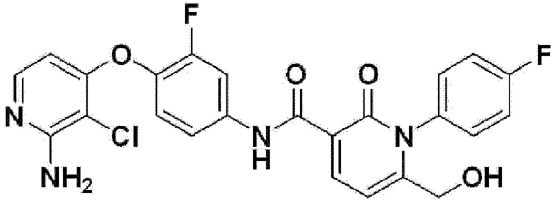

The title compound (24 mg, yield: 45%) was obtained in a manner similar to that of Example 24 using 1-(4-fluorophenyl)-6-(hydroxymethyl)-2-oxo-1,2-dihydropyridine-3-carboxylic acid instead of 1,5-dimethyl-3-oxo-2-phenyl-2,3-dihydro-1H-pyrazole-4-carboxylic acid according to Step 6 of Example 24.

¹H NMR (400 MHz, DMSO-d₆) δ 12.04 (s, 1H), 8.63 (d, 1H), 8.01 (m, 1H), 7.76 (d, 1H), 7.47 (m, 5H), 7.32 (t, 1H), 6.89 (d, 1H), 6.41 (brs, 2H), 5.95(d, 1H), 4.02(s, 2H); MS m/z : 499[M+H]

### Example 27. N-(4-((2-amino-3-chloropyridin-4-yl)oxy)-3-fluorophenyl)-5-cyclopropyl-1-(4-fluorophenyl)-2-oxo-1,2-dihydropyridine-3-carboxamide

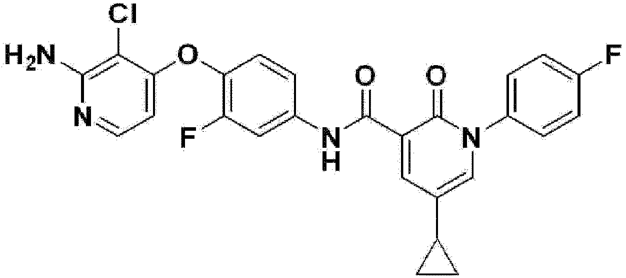

The title compound (30 mg, yield: 80%) was obtained in a manner similar to that of Example 24 using 5-cyclopropyl-1-(4-(fluorophenyl)-2-oxo-1,2-dihydropyridine-3-carboxylic acid instead of 1,5-dimethyl-3-oxo-2-phenyl-2,3-dihydro-1H-pyrazole-4-carboxylic acid according to Step 6 of Example 24.

¹H NMR (400 MHz, DMSO-d₆) δ 12.20 (s, 1H), 8.30 (d, 1H), 8.01 (m, 2H), 7.77 (d, 1H), 7.60 (m, 2H), 7.42 (m, 3H), 7.31 (m, 1H), 6.73 (d, 1H), 6.41 (brs, 2H), 5.96(d, 1H), 2.00(m, 1H), 0.93 (m, 2H), 0.72 (m, 2H); MS m/z : 509[M+H]

### Example 28. N-(4-((2-amino-3-chloropyridin-4-yl)oxy)-3-fluorophenyl)-1-(4-fluorophenyl)-2-oxo-6-(trifluoromethyl)-1,2-dihydropyridine-3-carboxamide

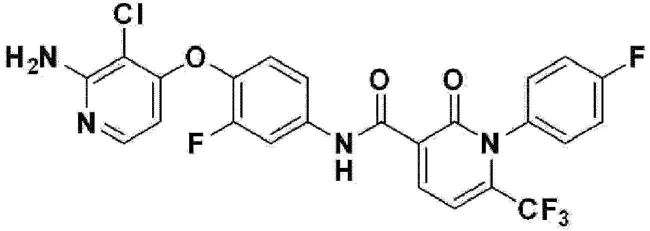

The title compound (48 mg, yield: 89%) was obtained in a manner similar to that of Example 24 using 1-(4-fluorophenyl)-2-oxo-6-(trifluoromethyl)-1,2-dihydropyridine-3-carboxylic acid instead of 1,5-dimethyl-3-oxo-2-phenyl-2,3-dihydro-1H-pyrazole-4-carboxylic acid according to Step 6 of Example 24.

¹H NMR (400 MHz, DMSO-d₆) δ 11.71 (s, 1H), 8.65 (d, 1H), 8.01 (m, 1H), 7.70 (d, 1H), 7.58 (m, 3H), 7.42 (m, 2H), 7.31 (t, 2H), 6.41 (brs, 2H), 5.95(d, 1H); MS m/z : 537[M+H]

### Example 29. N-(4-((2-amino-3-chloropyridin-4-yl)oxy)-3-fluorophenyl)-1-(2-hydroxy-2-methylpropyl)-5-methyl-3-oxo-2-phenyl-2,3-dihydro-1H-pyrazole-4-carboxamide

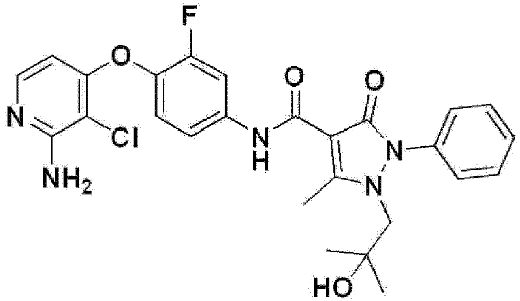

The title compound (39 mg, yield: 79%) was obtained in a manner similar to that of Example 24 using 1-(2-hydroxy-2-methylpropyl)-5-methyl-3-oxo-2-phenyl-2,3-dihydro-1H-pyrazole-4-carboxylic acid instead of 1,5-dimethyl-3-oxo-2-phenyl-2,3-dihydro-1H-pyrazole-4-carboxylic acid according to Step 6 of Example 24.

¹H NMR (400 MHz, MeOH-d₄) δ 8.16 (d, 1H), 7.91 (d, 1H), 7.73 (d, 1H), 7.62 (m, 2H), 7.58 (m, 1H), 7.39 (m, 2H), 7.28 (m, 1H), 7.18 (m, 1H), 6.01 (d, 1H), 3.97 (brs, 2H), 2.87 (s, 3H), 1.07 (s, 6H); MS m/z : 526[M+H]

### Example 30. N-(4-((3-chloro-2-(cyclopropanecarboxamido)pyridin-4-yl)oxy)-3-fluorophenyl)-1-(4-fluorophenyl)-6-methyl-2-oxo-1,2-dihydropyridine-3-carboxamide

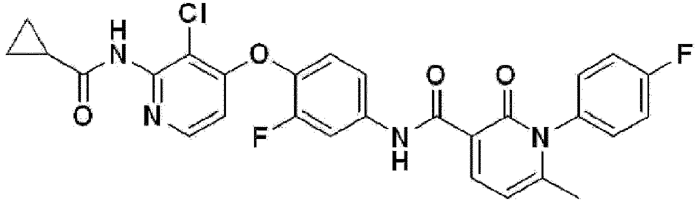

Hereinafter, a compound of Example 30 was prepared according to Reaction Scheme 10 below.

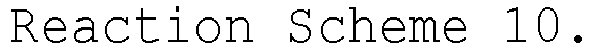

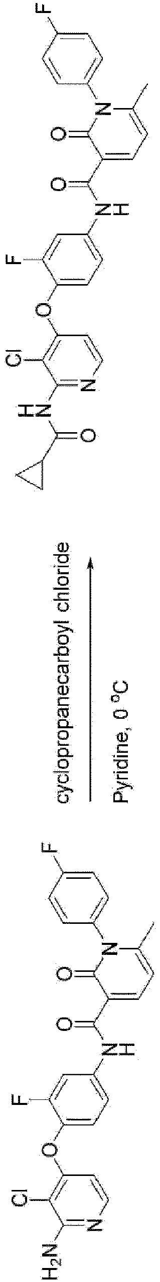

The compound N-(4-((2-amino-3-chloropyridin-4-yl)oxy)-3-fluorophenyl)-1-(4-fluorophenyl)-6-methyl-2-oxo-1,2-dihydropyridine-3-carboxamide (30 mg, 0.06 mmol) of Example 25 was dissolved in pyridine, and cyclopropanecarbonyl chloride (7 µL, 0.07 mmol) was slowly added thereto at 0 °C. The reaction mixture was stirred at 0 °C for 1 hour, diluted with EA, and washed with brine. The organic layer was concentrated, and then the residue was purified by prep-HPLC (0.1% formic acid in water/acetonitrile) to obtain the title compound (17 mg, yield: 49%).

¹H NMR (400 MHz, DMSO-d₆) δ 12.09 (s, 1H), 10.47 (s, 1H), 8.51 (d, 1H), 8.21 (d, 1H), 8.06 (d, 1H), 7.52 (m, 3H), 7.46 (m, 3H), 6.73 (m, 1H), 6.67 (d, 1H), 2.09 (s, 3H), 1.92 (m, 1H), 0.80 (m, 4H); MS m/z : 551[M+H]

### Example 31. N-(4-((3-chloro-2-(N-(cyclopropanecarbonyl)cyclopropanecarboxamido)pyridin-4-yl)oxy)-3-fluorophenyl)-1-(4-fluorophenyl)-6-methyl-2-oxo-1,2-dihydropyridine-3-carboxamide

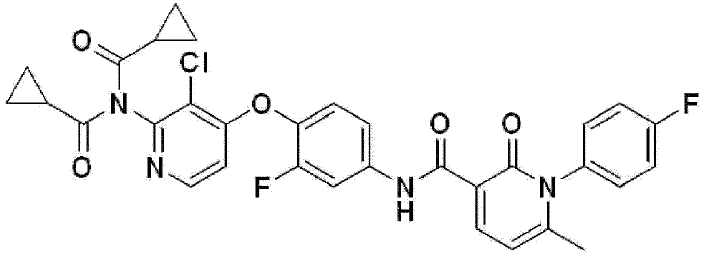

The title compound (7 mg, yield: 18%) was obtained as a by-product of Example 26.

¹H NMR (400 MHz, DMSO-d₆) δ 12.11 (s, 1H), 8.51 (d, 1H), 8.37 (d, 1H), 8.09 (d, 1H), 7.53 (m, 3H), 7.47 (m, 3H), 6.96 (m, 1H), 6.74 (d, 1H), 2.09 (s, 3H), 1.99 (m, 2H), 1.01 (m, 8H); MS m/z : 620[M+H]

### Example 32. 2-amino-5-bromo-N-(2-fluoro-4-(1-(4-fluorophenyl)-6-methyl-2-oxo-1,2-dihydropyridine-3-carboxamido)phenyl)nicotinamide

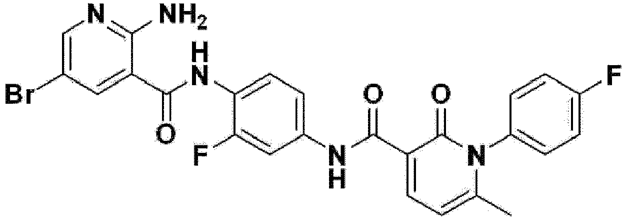

Hereinafter, a compound of Example 32 was prepared according to Reaction Scheme 11 below.

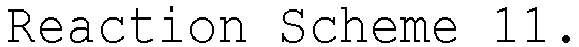

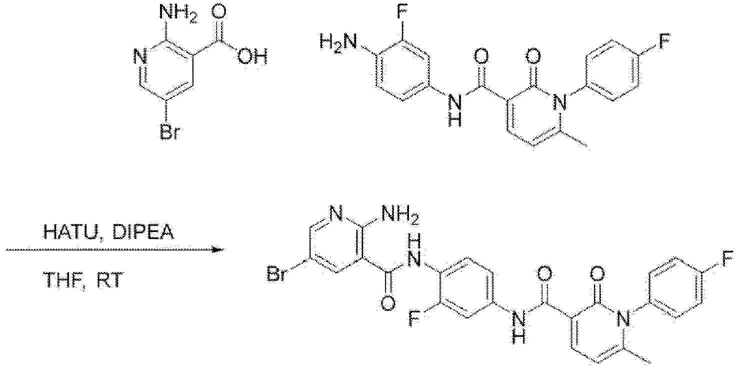

2-Amino-5-bromonicotinic acid (88mg, 0.41 mmol) was dissolved in DMF, and N-(4-amino-3-fluorophenyl)-4'-fluoro-6-methyl-2-oxo-1,2-dihydro-[1,1'-biphenyl]-3-carboxamide (120 mg, 0.34 mmol), HATU (193 mg, 0.51 mmol), and DIPEA (90 µL, 0.51 mmol) were added thereto. The reaction mixture was stirred at room temperature for about 12 hours. The reaction solution was diluted with water, and then extracted three times with EA and concentrated. The residue was purified by silica gel column chromatography to obtain the title compound (175 mg, 93%).

MS m/z : 553[M], 555[M+2]

### Example 33. 3-amino-6-bromo-N-(2-fluoro-4-(1-(4-fluorophenyl)-6-methyl-2-oxo-1,2-dihydropyridine-3-carboxamido)phenyl)pyrazine-2-carboxamide

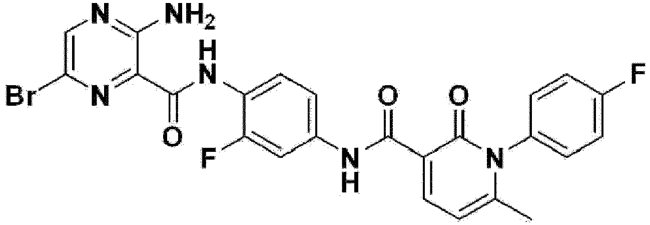

The title compound (135 mg, yield: 72%) was obtained in a manner similar to that of Example 32 using 3-amino-6-bromopyrazine-2-carboxylic acid instead of 2-amino-5-bromonicotinic acid according to Example 32.

MS *m*/*z* : 554[M], 556[M+2]

### Example 34. 2-amino-N-(2-fluoro-4-(1-(4-fluorophenyl)-6-methyl-2-oxo-1,2-dihydropyridin-3-carboxamido)phenyl)-5-(1-methyl-1H-pyrazol-4-yl)nicotinamide

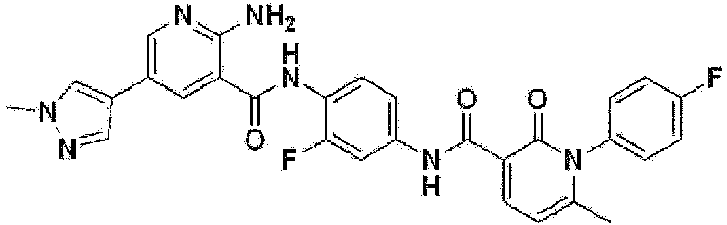

Hereinafter, a compound of Example 34 was prepared according to Reaction Scheme 12 below.

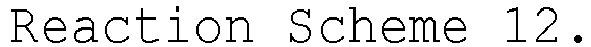

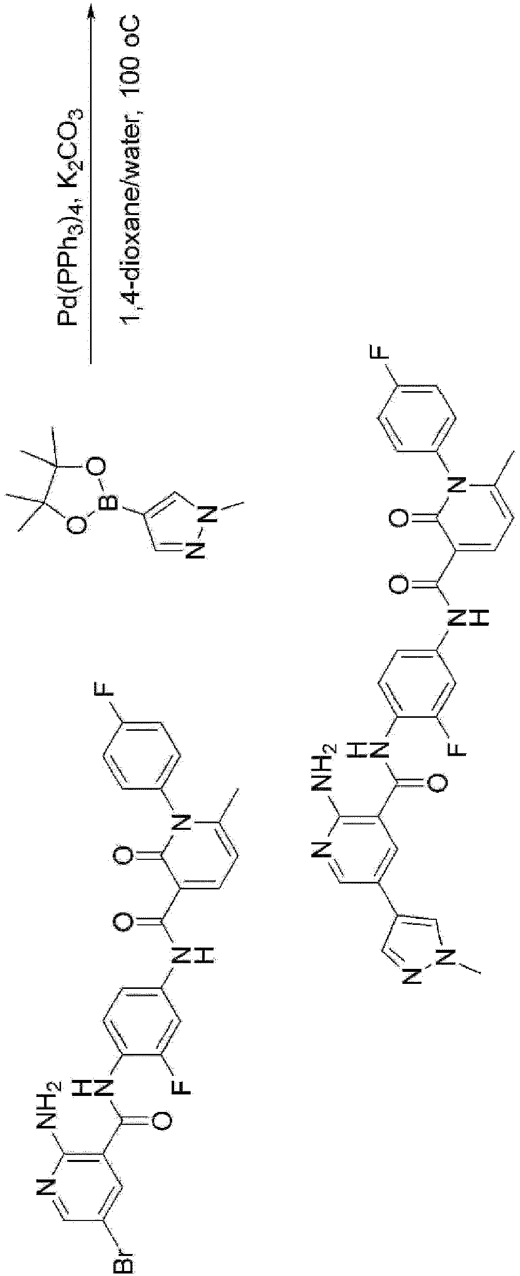

The compound 2-amino-5-bromo-N-(2-fluoro-4-(1-(4-fluorophenyl)-6-methyl-2-oxo-1,2-dihydropyridine-3-carboxamido)phenyl)nicotinamide (40 mg, 0.072 mmol) of Example 32 was dissolved in 1,4-dioxane/water (3:1), and 1-methyl-4-(4,4,5,5-tetramethyl-1,3,2-dioxaborolan-2-yl)-1H-pyrazol (45 mg, 0.22 mmol), K₂CO₃ (30 mg, 0.22 mmol), Pd(PPh₃)₄ (25 mg, 0.02 mmol) was added thereto, followed by stirring at 100 °C for 12 hours. The reaction mixture was diluted with water, extracted three times with DCM, and washed with brine and concentrated. The residue was purified by silica gel column chromatography to obtain the title compound (14 mg, yield: 34%).

¹H NMR (400 MHz, DMSO-d₆) δ 12.03 (s, 1H), 10.04 (s, 1H), 8.51 (d, 1H), 8.41 (s, 1H), 8.29 (s, 1H), 8.06 (d, 1H), 7.83 (d, 1H), 7.72 (s, 1H), 7.49 (m, 5H), 7.39 (m, 1H), 7.03 (brs, 2H), 6.73 (d, 1H), 3.87 (s, 3H), 2.09 (s, 3H); MS m/z : 556 [M+H]

### Example 35. 3-amino-N-(2-fluoro-4-(1-(4-fluorophenyl)-6-methyl-2-oxo-1,2-dihydropyridin-3-carboxamido)phenyl)-6-(1-methyl-1H-pyrazol-4-yl)pyrazine-2-carboxamide

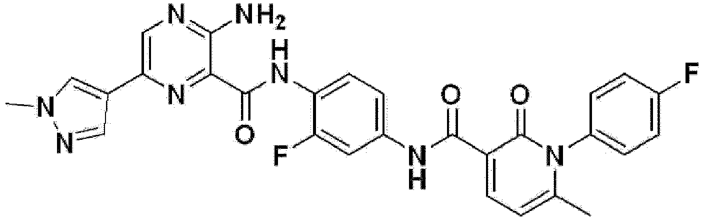

The title compound (9 mg, yield: 21%) was obtained in a similar manner to that of Example 34 using the compound 3-amino-6-bromo-N-(2-fluoro-4-(1-(4-fluorophenyl)-6-methyl-2-oxo-1,2-dihydropyridine-3-carboxamido)phenyl)pyrazine-2-carboxamide of Example 33 instead of 2-amino-5-bromo-N-(2-fluoro-4-(1-(4-fluorophenyl)-6-methyl-2-oxo-1,2-dihydropyridine-3-carboxamido)phenyl)nicotinamide of Example 34.

¹H NMR (400 MHz, DMSO-d₆) δ 12.05 (s, 1H), 10.26 (s, 1H), 8.67 (s, 1H), 8.50 (m, 1H), 8.38 (s, 1H), 8.12 (s, 1H), 7.96 (d, 1H), 7.76 (m, 1H), 7.52 (m, 6H), 6.73 (d, 2H), 3.90 (s, 3H), 2.09 (s, 3H); MS m/z : 557[M+H]

### Example 36. N-(4-((6-amino-5-chloropyrimidin-4-yl)oxy)-3-fluorophenyl)-1-(4-fluorophenyl)-6-methyl-2-oxo-1,2-dihydropyridine-3-carboxamide

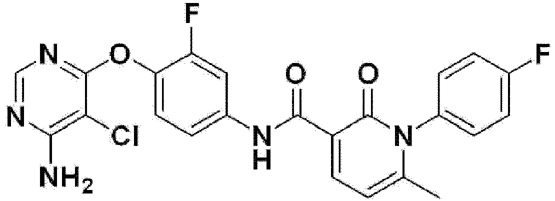

The title compound (32 mg, yield: 42%) was obtained in a manner similar to that of Example 25 using 6-(4-amino-2-fluorophenoxy)-5-chloropyrimidine-4-amine instead of 4-(4-amino-3-fluorophenoxy)-3-chloropyridine-2-amine according to Example 25.

¹H NMR (400 MHz, DMSO-d₆) δ 12.01 (s, 1H), 8.50 (d, 1H), 7.96 (s, 1H), 7.93 (d, 1H), 7.52 (m, 2H), 7.46 (m, 5H), 7.29 (t, 1H), 6.72 (d, 1H), 2.08 (s, 3H); MS m/z : 484[M+H]

### Experimental Example 1. Evaluation of inhibitory activity against RON and MET

### [1-1] Evaluation of inhibitory activity against RON

In order to measure the inhibitory activity of each of the compounds of Examples against RON, the IC50 of each of the compounds was measured using Time-resolved fluorescence energy transfer (TR-FRET).

Specifically, the compound was prepared to a concentration of 1 mM in 100% DMSO and was diluted 10 times in a stepwise manner through 3-fold dilution. The compound (2 µL) diluted in a stepwise manner was added to a 96-well-plate containing 48 µL of a 1x kinase reaction buffer (50 mM HEPES (pH 7.4), 0.01% Tween-20, 5 mM DTT, 0.5 mM Na₃VO₄, 2 mM EGTA, 10 mM MgCl₂).

The RON protein was diluted to a concentration of 49.3 nM in a RON storage buffer (50 mM Tris-HCl (pH 7.5), 150 mM NaCl, 0.5 mM EDTA, 0.02% Triton X-100), and then diluted again to a concentration of 0.4 nM in a 1x kinase reaction buffer. As a substrate cocktail, a RON-specific substrate mixed solution (40uM ULight-labeled Poly GT, 100 nM ATP) was prepared at a concentration 2 times the final reaction concentration.

Thereafter, a 384-well-plate was prepared, and 2.5 µL of each of the diluted compounds of Examples was added to a corresponding well of experimental group wells, and 2.5 µL of a 4% DMSO solution was dispensed into high control wells and low control wells.

Thereafter, 2.5 µL of 0.4 nM RON was dispensed into each of the high control wells and the experimental group wells, and 2.5 µL of the 1x kinase reaction buffer was added to the low control wells, and all the wells were centrifuged at room temperature for 40 seconds at 1,000 RPM, and then incubated at room temperature for 20-30 minutes. Then, 5 µL of the substrate cocktail (2x) was dispensed into each well, and all the wells were centrifuged at room temperature for 40 seconds at 1,000 RPM, and then incubated at room temperature for 60 minutes. Thereafter, 5 µL of 30 mM EDTA was added to each well to terminate the reaction, and all the wells were then incubated again at room temperature for 5 minutes. Thereafter, 5 µL of a 4x phosphotyrosine antibody (Perkin Elmer) containing europium was added to each well, and all the wells were then incubated at room temperature for 60 minutes. After the incubation, the 384-well-plate was read with the Vison plate reader. In this case, the excitation wavelength was 340 nm, and the emission wavelength was 620 nm and 665 nm. The IC50 value of each compound of Examples was derived using the GraphPd Prism7 program.

### [1-2] Evaluation of inhibitory activity against MET

In addition, in order to measure the enzyme activity inhibition for cMET, the evaluation was performed in the same manner as the enzyme activity inhibition measurement experiment for RON.

Specifically, the compound was prepared to a concentration of 1 mM in 100% DMSO and was diluted 10 times in a stepwise manner through 3-fold dilution. The compound (2 µL) diluted in a stepwise manner was added to a 96-well-plate containing 48 µL of a 1x kinase reaction buffer (50 mM HEPES (pH 7.4), 0.05% BSA, 0.005 % Tween-20, 1 mM DTT, 0.5 mM MnCl₂, 20 mM MgCl₂).

The cMET protein was diluted to a concentration of 263 nM in a cMET storage buffer (50 mM Tris-HCl (pH 7.5), 150 mM NaCl, 0.05% Brij35, 1 mM DTT, 10% Glycerol), and then diluted again to a concentration of 2 nM in a 1x kinase reaction buffer. As a substrate cocktail, a MET-specific substrate mixed solution (5 µM TK peptide, 10 mM ATP) was prepared at a concentration 2 times the final reaction concentration.

Thereafter, a 384-well-plate was prepared, and 2.5 µL of each of the diluted compounds of Examples was added to a corresponding well of experimental group wells, and 2.5 µL of a 4% DMSO solution was dispensed into high control wells and low control wells.

Thereafter, 2.5 µL of 2 nM cMET was dispensed into each of the high control wells and the experimental group wells, and 2.5 µL of the 1x kinase reaction buffer was added to the low control wells, and all the wells were centrifuged at room temperature for 40 seconds at 1,000 RPM, and then incubated at room temperature for 20-30 minutes. Then, 5 µL of the substrate cocktail (2x) was dispensed into each well, and all the wells were centrifuged at room temperature for 40 seconds at 1,000 RPM, and then incubated at room temperature for 60 minutes. Thereafter, 5 µL of 90 mM EDTA was added to each well to terminate the reaction, and all the wells were then incubated again at room temperature for 5 minutes. Thereafter, 5 µL of a 4x phosphotyrosine antibody (Perkin Elmer) containing europium was added to each well, and all the wells were then incubated at room temperature for 60 minutes. After the incubation, the 384-well-plate was read with the Vison plate reader. In this case, the excitation wavelength was 340 nm, and the emission wavelength was 620 nm and 665 nm. The IC50 value of each compound of Examples was derived using the GraphPd Prism7 program.

The results of evaluating the inhibitory activity of the compounds of Examples for RON or cMET are shown in Table 2 below.

**[Table 2]**

| | RON (IC₅₀, nM) | MET (IC₅₀, nM) |
|---|---|---|
| **Example 1** | 5,120 | 5,230 |
| **Example 3** | - | 6,720 |
| **Example 6** | - | 8,270 |
| **Example 15** | - | 4,857 |
| **Example 16** | - | 1,486 |
| **Example 18** | 467 | 496 |
| **Example 19** | - | 6,543 |
| **Example 21** | - | 6,033 |
| **Example 24** | 44 | 11 |
| **Example 25** | 1 | 2 |
| **Example 26** | 3 | 3 |
| **Example 27** | 2 | 6 |
| **Example 28** | 8 | 18 |
| **Example 29** | 82 | 54 |
| **Example 30** | 25 | 12 |
| **Example 31** | - | 3,551 |
| **Example 32** | - | 1,692 |
| **Example 34** | 7,880 | 1,559 |
| **Example 36** | 7 | 14 |

## Claims

1. A compound of Formula 1 below or a pharmaceutically acceptable salt thereof: 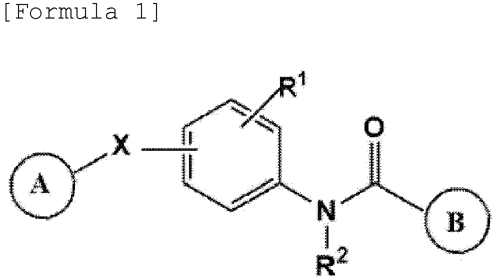 wherein, in Formula 1 above,
X above is a direct linkage, O, NR³ or NHCO,
A above is a 5- to 15-membered substituted or unsubstituted heteroaryl containing 1 to 5 hetero ring atoms selected from the group consisting of nitrogen, sulfur, and oxygen, wherein a substituent of the substituted heteroaryl is C₁-C₆ alkyl, halogen, amine, oxo, or C₂-C₆ cycloalkyl carboxamido,
A above is a single ring of the substituted or unsubstituted heteroaryl defined above, or a multi-ring in which two or more rings of the substituted or unsubstituted heteroaryl are fused or are connected by a carbon (sp²)-carbon (sp²) bond,
B above is a 5- to 7-membered substituted or unsubstituted heteroaryl containing an oxo group and containing 1 to 5 hetero ring atoms selected from the group consisting of nitrogen, sulfur and oxygen, wherein a substituent of the substituted heteroaryl is halogen, C₁-C₆ alkyl, hydroxy substituted C₁-C₆ alkyl, halogen substituted C₁-C₆ alkyl, C₁-C₆ alkyl substituted C₁-C₆ alkyl, C₁-C₆ alkoxy, 5-to 12-membered aryl, or halogen substituted 5- to 12-membered aryl,
R¹ above is hydrogen, C₁-C₆ alkyl or halogen,
R² above is hydrogen or C₁-C₆ alkyl,
R³ above is hydrogen or C₁-C₆ alkyl, and
the halogens are each independently selected from the group consisting of F, Cl, Br, and I.

2. The compound of claim 1 or a pharmaceutically acceptable salt thereof, wherein A above is a 5- to 12-membered substituted or unsubstituted heteroaryl containing 1 to 3 hetero ring atoms selected from nitrogen and sulfur, wherein the substituent of the substituted heteroaryl is methyl, halogen, amine, oxo or C₂-C₆ cyclopropane carboxamido.

3. The compound of claim 1 or a pharmaceutically acceptable salt thereof, wherein A above has a structure below:

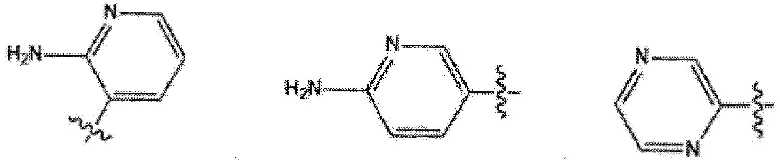

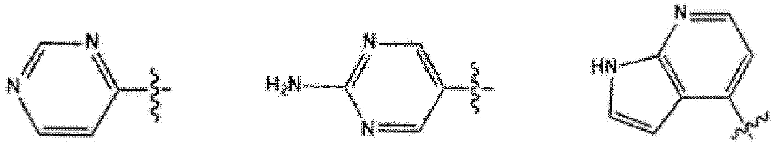

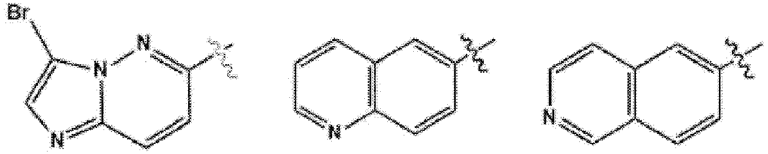

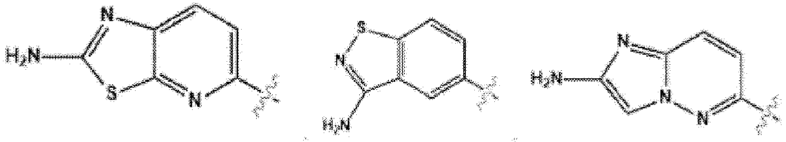

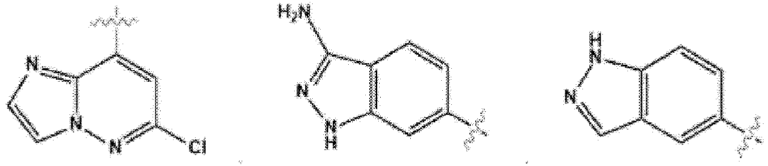

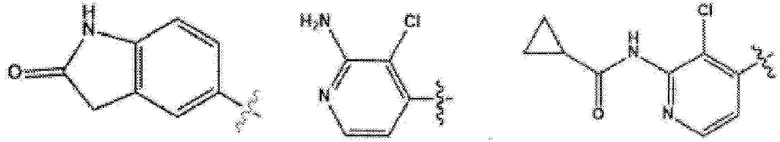

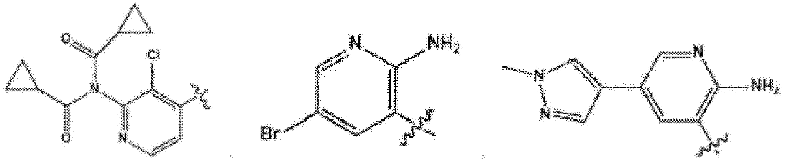

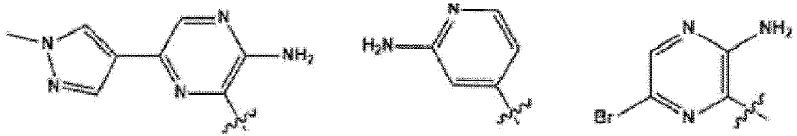

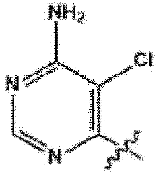

4. The compound of claim 1 or a pharmaceutically acceptable salt thereof, wherein B above has a structure of Formula 2 below or Formula 3 below: 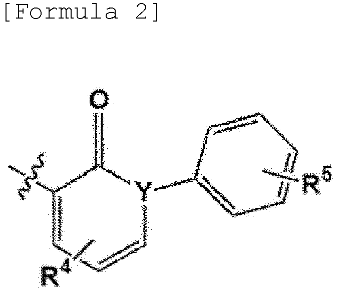 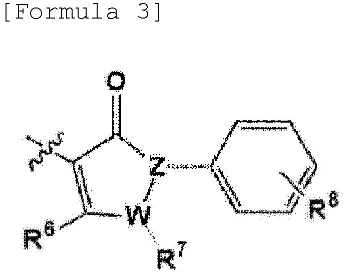 wherein, in Formula 2 or Formula 3 above,
Y, Z, and W above are each independently a heteroatom selected from the group consisting of nitrogen, sulfur, and oxygen,
R⁴, R⁶, and R⁷ above are each independently hydrogen, C₁-C₆ alkyl, halogen, hydroxy or halogen substituted C₁-C₆ alkyl, or C₁-C₆ alkoxy,
R₅ and R₈ above are each independently hydrogen or halogen.

5. The compound of claim 1 or a pharmaceutically acceptable salt thereof, wherein X above is a direct linkage, O, NH, or NHCO.

6. The compound of claim 1 or a pharmaceutically acceptable salt thereof, wherein R¹ above is hydrogen, methyl, or fluorine.

7. The compound of claim 1 or a pharmaceutically acceptable salt thereof, wherein R² above is hydrogen or methyl.

8. The compound of claim 1 or a pharmaceutically acceptable salt thereof, wherein the compound is selected from the group consisting of N-(4-(2-aminothiazolo[5,4-b]pyridin-5-yl)-3-methylphenyl)-4-ethoxy-1-(4-fluorophenyl)-2-oxo-1,2-dihydropyridine-3-carboxamide, N-(4-(2-aminothiazolo[5,4-b]pyridin-5-yl)-3-fluorophenyl)-4-ethoxy-1-(4-fluorophenyl)-2-oxo-1,2-dihydropyridine-3-carboxamide, N-(4-((2-aminothiazolo[5,4-b]pyridin-5-yl)amino)-3-fluorophenyl)-4-ethoxy-1-(4-fluorophenyl)-2-oxo-1,2-dihydropyridine-3-carboxamide, N-(4-(2-aminoimidazo[1,2-b]pyridazin-6-yl)-3-fluorophenyl)-4-ethoxy-1-(4-fluorophenyl)-2-oxo-1,2-dihydropyridine-3-carboxamide, N-(4-((6-chloroimidazo[1,2-b]pyridazin-8-yl)oxy)-3-fluorophenyl)-4-ethoxy-1-(4-fluorophenyl)-2-oxo-1,2-dihydropyridine-3-carboxamide, N-(4-((6-chloroimidazo[1,2-b]pyridazin-8-yl)oxy)-3-fluorophenyl)-1-(4-fluorophenyl)-6-methyl-2-oxo-1,2-dihydropyridine-3-carboxamide, N-(3-(6-aminopyridin-3-yl)-4-methylphenyl)-4-ethoxy-1-(4-fluorophenyl)-2-oxo-1,2-dihydropyridine-3-carboxamide, N-(3-(2-aminopyrimidin-5-yl)-4-methylphenyl)-4-ethoxy-1-(4-fluorophenyl)-2-oxo-1,2-dihydropyridine-3-carboxamide, N-(3-(2-aminopyridin-4-yl)-4-methylphenyl)-4-ethoxy-1-(4-fluorophenyl)-2-oxo-1,2-dihydropyridine-3-carboxamide, N-(3-(2-aminopyridin-3-yl)-4-methylphenyl)-4-ethoxy-1-(4-fluorophenyl)-2-oxo-1,2-dihydropyridine-3-carboxamide, 4-ethoxy-1-(4-fluorophenyl)-N-(4-methyl-3-(quinolin-6-yl)phenyl)-2-oxo-1,2-dihydropyridine-3-carboxamide, 4-ethoxy-1-(4-fluorophenyl)-N-(3-(isoquinolin-6-yl)-4-methylphenyl)-2-oxo-1, 2-dihydropyridine-3-carboxamide, 4-ethoxy-1-(4-fluorophenyl)-N-(4-methyl-3-(1H-pyrrolo[2,3-b]pyridin-4-yl)phenyl)-2-oxo-1,2-dihydropyridine-3-carboxamide, N-(4-((3-bromoimidazo[1,2-b]pyridazin-6-yl)oxy)-2-fluorophenyl)-4-ethoxy-1-(4-fluorophenyl)-2-oxo-1,2-dihydropyridine-3-carboxamide, N-(4-(2-(2-aminopyrimidin-4-yl)pyridin-4-yl)-3-fluorophenyl)-1-(4-fluorophenyl)-4-methoxy-2-oxo-1,2-dihydropyridine-3-carboxamide, N-(4-(2-(2-aminopyrimidin-4-yl)pyridin-4-yl)-3-fluorophenyl)-1-(4-fluorophenyl)-6-methyl-2-oxo-1,2-dihydropyridine-3-carboxamide, N-(4-(2-(2-aminopyrimidin-4-yl)pyridin-4-yl)-3-fluorophenyl)-5-bromo-1-(4-fluorophenyl)-2-oxo-1,2-dihydropyridine-3-carboxamide, N-(4-(3-amino-1H-indazol-6-yl)-3-fluorophenyl)-4-ethoxy-1-(4-fluorophenyl)-2-oxo-1,2-dihydropyridine-3-carboxamide, 4-ethoxy-N-(3-fluoro-4-(1H-indazol-5-yl)phenyl)-1-4-fluorophenyl)-2-oxo-1, 2-dihydropyridine-3-carboxamide, 4-ethoxy-N-(3-fluoro-4-(2-oxoindolin-5-yl)phenyl)-1-(4-fluorophenyl)-2-oxo-1,2-dihydropyridine-3-carboxamide, N-(4-(3-aminobenzo[d]isothiazol-5-yl)-3-fluorophenyl)-4-ethoxy-1-(4-fluorophenyl)-2-oxo-1,2-dihydropyridine-3-carboxamide, N-(4-(3-amino-1H-indazol-6-yl)-3-fluorophenyl)-1-(2-hydroxy-2-methylpropyl)-5-methyl-3-oxo-2-phenyl-2,3-dihydro-1H-pyrazole-4-carboxamide, N-(4-(3-amino-1H-indazol-6-yl)-3-fluorophenyl)-1-(4-fluorophenyl)-6-methyl-2-oxo-1,2-dihydropyridine-3-carboxamide, N-(4-((2-amino-3-chloropyridin-4-yl)oxy)-3-fluorophenyl)-1,5-dimethyl-3-oxo-2-phenyl-2,3-dihydro-1H-pyrazole-4-carboxamide, N-(4-((2-amino-3-chloropyridin-4-yl)oxy)-3-fluorophenyl)-1-(4-fluorophenyl)-6-methyl-2-oxo-1,2-dihydropyridine-3-carboxamide, N-(4-((2-amino-3-chloropyridin-4-yl)oxy)-3-fluorophenyl)-1-(4-fluorophenyl)-6-(hydroxymethyl)-2-oxo-1,2-dihydropyridine-3-carboxamide, N-(4-((2-amino-3-chloropyridin-4-yl)oxy)-3-fluorophenyl)-5-cyclopropyl-1-(4-fluorophenyl)-2-oxo-1,2-dihydropyridine-3-carboxamide, N-(4-((2-amino-3-chloropyridin-4-yl)oxy)-3-fluorophenyl)-1-(4-fluorophenyl)-2-oxo-6-(trifluoromethyl)-1,2-dihydropyridine-3-carboxamide, N-(4-((2-amino-3-chloropyridin-4-yl)oxy)-3-fluorophenyl)-1-(2-hydroxy-2-methylpropyl)-5-methyl-3-oxo-2-phenyl-2,3-dihydro-1H-pyrazole-4-carboxamide, N-(4-((3-chloro-2-(cyclopropanecarboxamido)pyridin-4-yl)oxy)-3-fluorophenyl)-1-(4-fluorophenyl)-6-methyl-2-oxo-1,2-dihydropyridine-3-carboxamide, N-(4-((3-chloro-2-(N-(cyclopropanecarbonyl)cyclopropanecarboxamido)pyridin-4-yl)oxy)-3-fluorophenyl)-1-(4-fluorophenyl)-6-methyl-2-oxo-1,2-dihydropyridine-3-carboxamide, 2-amino-5-bromo-N-(2-fluoro-4-(1-(4-fluorophenyl)-6-methyl-2-oxo-1,2-dihydropyridin-3-carboxamido)phenyl)nicotinamide, 3-amino-6-bromo-N-(2-fluoro-4-(1-(4-fluorophenyl)-6-methyl-2-oxo-1,2-dihydropyridine-3-carboxamido)phenyl)pyrazine-2-carboxamide, 2-amino-N-(2-fluoro-4-(1-(4-fluorophenyl)-6-methyl-2-oxo-1,2-dihydropyridin-3-carboxamido)phenyl)-5-(1-methyl-1H-pyrazol-4-yl)nicotinamide, 3-amino-N-(2-fluoro-4-(1-(4-fluorophenyl)-6-methyl-2-oxo-1,2-dihydropyridin-3-carboxamido)phenyl)-6-(1-methyl-1H-pyrazol-4-yl)pyrazine-2-carboxamide and N-(4-((6-amino-5-chloropyrimidin-4-yl)oxy)-3-fluorophenyl)-1-(4-fluorophenyl)-6-methyl-2-oxo-1,2-dihydropyridine-3-carboxamide.

9. A pharmaceutical composition comprising: the compound according to any one of claims 1 to 8 or a pharmaceutically acceptable salt thereof; and a pharmaceutically acceptable carrier.
